(19) Europäisches Patentamt
European Patent Office
Office européen des brevets

(11) Publication number: 0 468 683 B1

(12) EUROPEAN PATENT SPECIFICATION

(45) Date of publication of patent specification: 14.06.95

(51) Int. Cl.[6]: C07C 237/42, C07D 205/08, C07D 295/18, C07C 327/38, C07C 271/28, C07C 275/42, C07C 323/52, C07C 317/44, A01N 37/46, A01N 53/00, A01N 43/44, //A01N47/20, A01N47/30

(21) Application number: 91306446.5

(22) Date of filing: 16.07.91

The file contains technical information submitted after the application was filed and not included in this specification

(54) **Fungicidal acylamidobenzamides.**

(30) Priority: 27.07.90 GB 9016581

(43) Date of publication of application: **29.01.92 Bulletin 92/05**

(45) Publication of the grant of the patent: **14.06.95 Bulletin 95/24**

(84) Designated Contracting States: **AT BE CH DE DK ES FR GB GR IT LI LU NL SE**

(56) References cited:
EP-A- 0 002 309
EP-A- 0 127 990
EP-A- 0 381 330
FR-A- 2 369 263

(73) Proprietor: **ZENECA LIMITED**
**15 Stanhope Gate**
**London W1Y 6LN (GB)**

(72) Inventor: **Glen, Alasdair Thomas**
**5 Hall Grove**
**Macclesfield,**
**Cheshire SK10 2HO (GB)**
Inventor: **Spence, Rosamund Alison**
**Chazey Court Farm,**
**The Warren**
**Caversham,**
**Berkshire RG4 7TO (GB)**
Inventor: **Lawson, Kevin Robert**
**High Stile,**
**Piddington Lane**
**Piddington,**
**High Wycombe,**
**Bucks HP14 3BB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 468 683 B1

Rank Xerox (UK) Business Services
(3.10/3.09/3.3.3)

**CHEMICAL ABSTRACTS, vol. 92, no. 3, 21 January 1980 Columbus, Ohio, USA HASSAN K. M. ET AL.: "STUDIES ON LACTAMS AND THIAZOLIDINON & INDIAN J. CHEM. SECT. B 1979 18B[1] 44-6 page 682; left-hand column; ref. no. 22465G**

**PATENT ABSTRACTS OF JAPAN vol. 6, no. 40 (C-94)(918) 12 March 1982, & JP-A-56 156246 (HODOGAYA KAGAKU KOGYO K.K.) 02 December 1981,**

Inventor: **Crowley, Patrick Jelf**
**56 Ellis Avenue**
**Crowthorne,**
**Berkshire (GB)**

(74) Representative: **Tierney, Francis John et al**
**Intellectual Property Department,**
**ZENECA Agrochemicals,**
**Jealotts Hill Research Station,**
**P.O. Box 3538**
**Bracknell,**
**Berkshire RG12 6YA (GB)**

## Description

This invention relates to novel fungicidal acylaminobenzamides, to processes for preparing them, to fungicidal compositions containing them and to methods of using them to combat fungi, especially fungal infections of plants.

Acknowledgement is made of EP-A-0127990 and of US-A-4282218 which, for example, claims priority of UK Application No. 42454/77. The former describes acylanilides which have antiandrogenic properties and the latter describes aniline derivatives which have fungicidal properties.

According to the present invention there is provided a compound of the formula (I):

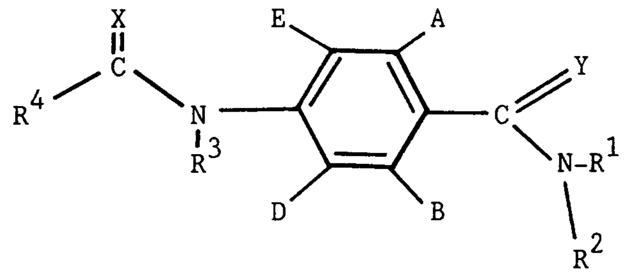

(I)

in which A and B are independently H, iodo, nitro, cyano, $C_{1-4}$ alkoxycarbonyl, $C_{1-4}$ alkoxy($C_{1-4}$)alkyl, $C_{1-4}$ alkylthio($C_{1-4}$)alkyl, formyl, $C_{1-4}$ alkylthio, $C_{1-4}$alkylcarbonyl, $-CR^5=NOR^6$ (where $R^5$ and $R^6$ are independently H or $C_{1-4}$ alkyl), $C_{2-4}$ alkenyl or $C_{2-4}$ alkynyl, provided that A and B are not both H; D and E are independently H or fluoro; $R^1$ is $C_{1-4}$ alkyl or $C_{1-4}$ alkoxy; $R^2$ is $C_{1-4}$ alkyl or $C_{1-4}$ alkoxy, or $R^1$ and $R^2$ together with the nitrogen atom to which they are attached join to form a morpholine, piperidine, pyrrolidine or azetidine ring which is optionally substituted with $C_{1-4}$ alkyl; $R^3$ is H; $R^4$ is $R(CH_3)_2C$, where R is halo, $C_{1-4}$ alkyl or $C_{1-4}$ alkoxy; and X and Y are independently oxygen or sulphur.

Alkyl groups and the alkyl moiety of other alkyl-containing groups can be in the form of straight or branched chains. Examples are methyl, ethyl, propyl (n-and iso-propyl), butyl (n-, sec, iso- and t-butyl), 1,1-dimethylpropyl and 1,1-dimethylbutyl. Alkenyl and alkynyl groups can also be in the form of straight or branched chains. Examples are 1,1-dimethylbut-3-enyl and 1,1-dimethylprop-2-ynyl.

$R^4$ is especially the group $F(CH_3)_2C$.

Halogen includes fluorine, chlorine and bromine.

Optional substituents of phenyl include: halogen, $C_{1-4}$ alkyl (for example, methyl), $C_{1-4}$ alkoxy (for example, methoxy), $C_{1-4}$ alkylthio (for example, methylthio), trifluoromethyl, trifluoromethoxy, nitro, cyano, $C_{1-4}$ alkoxycarbonyl, amino and mono- and di($C_{1-4}$)alkylamino.

In one aspect the invention provides a compound of the formula (I) in which A is iodo, cyano, nitro, $C_{1-4}$ alkoxycarbonyl, $C_{1-4}$ alkylcarbonyl, $C_{1-4}$ alkoxy($C_{1-4}$)alkyl, $C_{1-4}$ alkylthio, formyl, $C_{1-4}$ alkylthio-($C_{1-4}$)alkyl or $-CH=NOR^6$ (where $R^6$ is $C_{1-4}$ alkyl); $R^1$ and $R^2$ are alkyl (especially methyl or ethyl); $R^3$, B, D and E are H; $R^4$ is $C_{2-6}$ alkyl optionally substituted with halogen or $C_{1-4}$ alkoxy; and X and Y are both oxygen.

The invention is illustrated by the compounds listed in Table I which follows. These compounds have the formula (I) in which $R^1$, $R^2$, $R^4$, A and B have the values shown in the table. $R^3$, D and E are H, and X and Y are O.

TABLE I

| Compound No | $R^1$ | $R^2$ | $R^4$ | A | B | mpt (°C) |
|---|---|---|---|---|---|---|
| 1 | $CH_3$ | $CH_3$ | $(CH_3)_3C$ | I | H | 228.5-230.5 |
| 2 | $CH_3$ | $CH_3$ | $CH_3CH_2(CH_3)_2C$ | I | H | |
| 3 | $CH_3$ | $CH_3$ | $Cl(CH_3)_2C$ | I | H | |
| 4 | $CH_3$ | $CH_3$ | $Br(CH_3)_2C$ | I | H | |
| 5 | $CH_3$ | $CH_3$ | $F(CH_3)_2C$ | I | H | 165.5-168 |
| 6 | $CH_3$ | $CH_3$ | $CH_3O(CH_3)_2C$ | I | H | |
| 7 | $CH_3$ | $CH_3CH_2$ | $(CH_3)_3C$ | I | H | |
| 8 | $CH_3$ | $CH_3CH_2$ | $CH_3CH_2(CH_3)_2C$ | I | H | |
| 9 | $CH_3$ | $CH_3CH_2$ | $Cl(CH_3)_2C$ | I | H | |
| 10 | $CH_3$ | $CH_3CH_2$ | $Br(CH_3)_2C$ | I | H | |
| 11 | $CH_3$ | $CH_3CH_2$ | $F(CH_3)_2C$ | I | H | |
| 12 | $CH_3$ | $CH_3CH_2$ | $CH_3O(CH_3)_2C$ | I | H | |
| 13 | $CH_3$ | $CH_3$ | $(CH_3)_3C$ | CN | H | 145-148.5 |
| 14 | $CH_3$ | $CH_3$ | $F(CH_3)_2C$ | CN | H | |
| 15 | $CH_3$ | $CH_3$ | $(CH_3)_3C$ | $NO_2$ | H | 189-194 |

TABLE I (Continued)

| Compound No | $R^1$ | $R^2$ | $R^4$ | A | B | mpt (°C) |
|---|---|---|---|---|---|---|
| 16 | $CH_3$ | $CH_3$ | $(CH_3)_3C$ | $COOCH_3$ | H | 260 (decomp) |
| 17 | $CH_3$ | $CH_3$ | $(CH_3)_3C$ | $COCH_3$ | H | |
| 18 | $CH_3$ | $CH_3$ | $(CH_3)_3C$ | $CH_2OCH_3$ | H | |
| 19 | $CH_3$ | $CH_3$ | $(CH_3)_3C$ | $SCH_3$ | H | * |
| 20 | $CH_3$ | $CH_3$ | $F(CH_3)_2C$ | $COOCH_3$ | H | |
| 21 | $CH_3$ | $CH_3$ | $F(CH_3)_2C$ | $COCH_3$ | H | |
| 22 | $CH_3$ | $CH_3$ | $F(CH_3)_2C$ | $CH_2OCH_3$ | H | |
| 23 | $CH_3$ | $CH_3$ | $F(CH_3)_2C$ | $SCH_3$ | H | * |
| 24 | $CH_3$ | $CH_3$ | $(CH_3)_3C$ | CHO | H | |
| 25 | $CH_3$ | $CH_3$ | $(CH_3)_3C$ | $CH_2SCH_3$ | H | |
| 26 | $CH_3$ | $CH_3$ | $(CH_3)_3C$ | $CH{=}NOCH_3$ | H | |
| 28 | $CH_3$ | $CH_3$ | $(CH_3)_3C$ | $COOCH_2CH_3$ | H | 208-209 |
| 29 | $CH_3$ | $CH_3$ | $(CH_3)_3C$ | CHO | H | 186-187 |
| 33 | $CH_3$ | $CH_3$ | $F(CH_3)_2C$ | $-C\equiv CH$ | H | 110(decomp) |
| 34 | $CH_3$ | $CH_3$ | $F(CH_3)_2C$ | $-HC{=}CH_2$ | H | 164-165 |
| 35 | $CH_3$ | $CH_3$ | $(CH_3)_3C$ | $-C\equiv CH$ | H | 130(decomp) |

*<u>Compound No. 19</u>

$^1H$ NMR (270MHz; $CDCl_3$) δ 1.33(9H,s), 2.50(3H,s), 2.85(3H,s), 3.10(3H,s), 7.13(2H,d), 7.40(1H,s), 7.74(1H,s) ppm.

*<u>Compound No. 23</u>

$^1H$ NMR (270MHz; $CDCl_3$) δ 1.68(6H,d), 2.50(3H,s), 2.85(3H,s), 3.12(3H,s), 7.18(1H,d), 7.25(1H,dd), 7.72(1H,d), 8.15(1H,bs) ppm.

The compounds of the invention can be made by, for example, the methods illustrated in Schemes 1 and 2. Throughout these Schemes $R^1$, $R^2$, $R^4$, A, B, D and E are as defined before.

In Scheme 1, compounds of formula (II) where A is for example cyano, or $C_{1-4}$ alkylthio, can be prepared by reacting iodo compounds of formula (IX) with a transition metal salt, for example cuprous cyanide, or cuprous methanethiolate, in a suitable solvent such as <u>N,N</u>-dimethylformamide or dimethylsulphoxide.

Iodo compounds of formula (IX) can be made from anilines of formula (VIII), by treatment with nitrous acid (generated for example from sodium nitrite and sulphuric acid) and an iodide source such as potassium iodide.

Anilines of formula (VIII) can be made from compounds of formula (VII), where P is a protecting group. For example, when P is a trifluoroacetyl group, it can be removed by treatment of compounds of formula (VII) with a base in a hydroxylic solvent, such as potassium carbonate in methanol.

Compounds of formula (VII) can be prepared from anilines of formula (VI) by reaction with an acid chloride $R^4COCl$ in a suitable organic solvent such as methylene chloride in the presence of a base such as a tertiary amine (for example triethylamine) or an alkali metal carbonate or hydroxide (for example sodium carbonate or sodium hydroxide).

Anilines of formula (VI) can be prepared from nitro compounds of formula (V), by reduction using standard methods, such as hydrogenation over a precious metal catalyst (for example palladium on charcoal) in a suitable solvent (for example ethyl acetate) or iron, or stannous chloride, in hydrochloric acid.

Nitro amides of formula (V) can be made from nitro acids of formula (IV), by treatment first with a standard reagent such as thionyl chloride or oxalyl chloride to give the acid chloride, which is then reacted with an amine $R^1R^2NH$ in a suitable solvent, such as methylene chloride, in the presence of a base such as a tertiary amine (for example triethylamine), or an alkali metal carbonate or hydroxide (for example potassium carbonate, or sodium hydroxide).

Nitro acids of formula (IV) can be made from compounds of formula (III) by protection of the amino group, with a protecting group P, such as trifluoroacetyl, by reaction with trifluoroacetic anhydride.

In Scheme 2, compounds of formula (II) can be prepared from amino amides of formula (X) by reaction with an acid chloride $R^4COCl$ in a suitable organic solvent such as dichloromethane in the presence of a base such as a tertiary amine (for example triethylamine) or an alkali metal carbonate or hydroxide (for example sodium carbonate or sodium hydroxide).

Amino amides of formula (X) can be prepared from nitro compounds of formula (XXVII) by reduction using standard methods, such as hydrogenation over a precious metal catalyst (for example palladium on charcoal) in a suitable solvent (for example ethyl acetate) or iron, or stannous chloride in hydrochloric acid.

The route described in Scheme 2 is particularly useful for preparing compounds of formula (II) where A is iodo, $C_{2-4}$ alken-l-yl or $C_{2-4}$ alkyn-l-yl. In the case where A is $C_{2-4}$ alken-l-yl or $C_{2-4}$ alkyn-l-yl, the nitro compounds of formula (XXVII) can be prepared from iodides of formula (XXVI) by reaction with a $C_{2-4}$alkyne or a 1-tri-n--butylstannyl-($C_{2-4}$)alkene in a suitable solvent (for example N,N-dimethylformamide or acetonitrile) in the presence of a catalyst (for example bis (triphenylphosphine)palladium-II-dichloride) optionally in the presence of a copper I salt (for example cuprous iodide). Where A is ethynyl, the $C_{2-4}$alkyne used is trimethylsilylethyne, and the trimethylsilyl group may be removed in a subsequent step by treatment with potassium carbonate in a suitable solvent such as methanol. In the case where A is iodo, the iodides of formula (XXVI) are used directly as the nitro compounds of formula (XXVII).

Iodides of formula (XXVI) can be prepared from nitro acids of formula (XXV) by treatment first with a standard reagent such as thionyl chloride or oxalyl chloride to give the acid chloride, which is then reacted with an amine $R^1R^2NH$ in a suitable solvent, such as dichloromethane, in the presence of a base such as a tertiary amine (for example triethylamine), or an alkali metal carbonate or hydroxide (for example potassium carbonate or sodium hydroxide).

Nitro acids of formula (XXV) can be prepared from anilines of formula (III) by treatment with nitrous acid (generated for example from sodium nitrite and sulphuric acid) and an iodide source such as potassium iodide.

The compounds of the invention may also be prepared using methods and techniques described in EP-A-0381330 and in UK Applications Nos. 9016577.0 CORRESPONDING TO EP-A-0468681, 9016580.4 CORRESPONDING TO EP-A-0468682 AND 9016582.0 CORRESPONDING TO EP-A-0470711 and applications claiming priority therefrom.

In further aspects, the invention provides processes as hereinbefore described for preparing the compounds of the invention and also the following processes:

a) when X and Y are both oxygen and $R^3$ is H,

i) by reacting a compound of general formula (X), with an acid chloride $R^4COCl$ in a suitable organic solvent in the presence of a base; or

ii) by reacting a compound of general formula (XI), with an amine $R^1R^2NH$ in a suitable organic solvent in the presence of a base or excess $R^1R^2NH$; or

iii) by reacting a compound of general formula (XII), with a compound of general formula $R^4$-CO-$NH_2$ and a base; or

b) when X and Y are both oxygen, $R^3$ is H and $R^4$ is the group (XV),

i) by treating a compound of general formula (XVI), with a fluoride transfer reagent in a suitable solvent; or

c) when X is oxygen or sulphur, Y is sulphur and $R^3$ is H,

i) by treating a compound of general formula (II), with a thionation reagent in a suitable solvent to form either a compound of general formula (I) in which X is oxygen, Y is sulphur and $R^3$ is hydrogen or a mixture of a compound of general formula (I) in which X is oxygen, Y is sulphur and $R^3$ is hydrogen

and a compound of general formula (I) in which X and Y are both sulphur and $R^3$ is hydrogen; or

d) when X is sulphur, Y is oxygen and $R^3$ is H,

i) by reacting an isothiocyanate of general formula (XXI), with an organometallic reagent $R^4Li$ or $R^4Mg$-hal in a suitable solvent at a temperature between -78 °C and +25 °C; or

ii) by reacting an acid chloride (XXII), with an amine $R^1R^2NH$ in the presence of a base;

wherein A, B, D, E, $R^1$, $R^2$ and $R^4$ (except where otherwise stated) have the meanings given in claim 1, $R^8$ and $R^9$ are both methyl, hal is halogen and L is a leaving group.

The compounds of the invention show fungicidal activity across a range of plant diseases. They are, however, particularly active against the class of pathogens known as the phycomycetes (equivalent to the oomycetes). These include species of Phytophthora, Plasmopara, Peronospora and Pseudoperonospora. Examples of pathogens which the invention compounds are particularly useful for controlling are: Plasmopara viticola on vines; other downy mildews such as Bremia lactucae on lettuce; Peronospora spp. on soybeans, tobacco, onions and other hosts; Pseudoperonospora humuli on hops and Pseudoperonospora cubensis on cucurbits; Phytophthora infestans on potatoes and tomatoes and other Phytophthora spp. on vegetables, strawberries, avocado, pepper, ornamentals, tobacco, cocoa and other hosts; and Pythium sp on rice, horticultural plants, vegetables and turf.

The invention therefore provides a method of combating fungi which comprises applying to a plant, to a seed of a plant or to the locus of the plant or seed a fungicidally effective amount of a compound as hereinbefore defined, or a composition containing the same.

The compounds may be used directly for agricultural purposes but are more conveniently formulated into compositions using a carrier or diluent. The invention thus provides fungicidal compositions comprising a compound as hereinbefore defined and an acceptable carrier or diluent therefor.

The compounds can be applied in a number of ways. For example, they can be applied, formulated or unformulated, directly to the foliage of a plant, to seeds or to other medium in which plants are growing or are to be planted, or they can be sprayed on, dusted on or applied as a cream or paste formulation, or they can be applied as a vapour or as slow release granules.

Application can be to any part of the plant including the foliage, stems, branches or roots, or to soil surrounding the roots, or to the seed before it is planted, or to the soil generally, to paddy water or to hydroponic culture systems. The invention compounds may also be injected into plants or sprayed onto vegetation using electrodynamic spraying techniques or other low volume methods.

The term "plant" as used herein includes seedlings, bushes and trees. Furthermore, the fungicidal method of the invention includes preventative, protectant, prophylactic and eradicant treatments.

The compounds are preferably used for agricultural and horticultural purposes in the form of a composition. The type of composition used in any instance will depend upon the particular purpose envisaged.

The compositions may be in the form of dustable powders or granules comprising the active ingredient (invention compound) and a solid diluent or carrier, for example, fillers such as kaolin, bentonite, kieselguhr, dolomite, calcium carbonate, talc, powdered magnesia, fuller's earth, gypsum, diatomaceous earth and china clay. Such granules can be preformed granules suitable for application to the soil without further treatment. These granules can be made either by impregnating pellets of filler with the active ingredient or by pelleting a mixture of the active ingredient and powdered filler. Compositions for dressing seed may include an agent (for example, a mineral oil) for assisting the adhesion of the composition to the seed; alternatively the active ingredient can be formulated for seed dressing purposes using an organic solvent (for example, N-methylpyrrolidone, propylene glycol or N,N-dimethylformamide). The compositions may also be in the form of wettable powders or water dispersible granules comprising wetting or dispersing agents to facilitate the dispersion in liquids. The powders and granules may also contain fillers and suspending agents.

Emulsifiable concentrates or emulsions may be prepared by dissolving the active ingredient in an organic solvent optionally containing a wetting or emulsifying agent and then adding the mixture to water which may also contain a wetting or emulsifying agent. Suitable organic solvents are aromatic solvents such as alkylbenzenes and alkylnaphthalenes, ketones such as cyclohexanone and methylcyclohexanone, chlorinated hydrocarbons such as chlorobenzene and trichlorethane, and alcohols such as benzyl alcohol, furfuryl alcohol, butanol and glycol ethers.

Suspension concentrates of largely insoluble solids may be prepared by ball or bead milling with a dispersing agent with a suspending agent included to stop the solid settling.

Compositions to be used as sprays may be in the form of aerosols wherein the formulation is held in a container under pressure of a propellant, e.g. fluorotrichloromethane or dichlorodifluoromethane.

The invention compounds can be mixed in the dry state with a pyrotechnic mixture to form a composition suitable for generating in enclosed spaces a smoke containing the compounds.

Alternatively, the compounds may be used in micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities. Other additives may be included to improve the biological efficacy of the various formulations. Such additives can be surface active materials to improve the wetting and retention on surfaces treated with the formulation and also the uptake and mobility of the active material, or additionally can include oil based spray additives. For example, certain mineral oil and natural plant oil (such as soya bean and rape seed oil) additives have been found to enhance several-fold foliar protectant activity against, for example, Plasmopara viticola.

The invention compounds can be used as mixtures with fertilizers (e.g. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising a fertilizer and the compound of general formula (I) or a salt or metal complex thereof.

Wettable powders, emulsifiable concentrates and suspension concentrates will normally contain surfactants, e.g. a wetting agent, dispersing agent, emulsifying agent or suspending agent. These agents can be cationic, anionic or non-ionic agents.

Suitable cationic agents are quaternary ammonium compounds, for example, cetyltrimethylammonium bromide. Suitable anionic agents are soaps, salts of aliphatic monoesters of sulphuric acid (for example, sodium lauryl sulphate), and salts of sulphonated aromatic compounds (for example, sodium dodecylbenzenesulphonate, sodium, calcium or ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonylphenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example, polyvinylpyrrolidone and sodium carboxymethylcellulose), and swelling clays such as bentonite or attapulgite.

Compositions for use as aqueous dispersions or emulsions are generally supplied in the form of a concentrate containing a high proportion of the active ingredient, the concentrate being diluted with water before use. These concentrates should preferably be able to withstand storage for prolonged periods and after such storage be capable of dilution with water in order to form aqueous preparations which remain homogeneous for a sufficient time to enable them to be applied by conventional spray equipment. The concentrates may conveniently contain up to 95%, suitably 10-85%, for example 25-60%, by weight of the active ingredient. After dilution to form aqueous preparations, such preparations may contain varying amounts of the active ingredient depending upon the intended purpose, but an aqueous preparation containing 0.0005% or 0.01% to 10% by weight of active ingredient may be used.

The compositions of this invention may contain other compounds having biological activity, e.g. compounds having similar or complementary fungicidal activity or which possess plant growth regulating, herbicidal or insecticidal activity.

A fungicidal compound which may be present in the composition of the invention may be one which is capable of combating ear diseases of cereals (e.g. wheat) such as Septoria, Gibberella and Helminthosporium spp., seed and soil-borne diseases and downy and powdery mildews on grapes and powdery mildew and scab on apple, etc. By including another fungicide, the composition can have a broader spectrum of activity than the compound of general formula (I) alone. Further the other fungicide can have a synergistic effect on the fungicidal activity of the compound of general formula (I). Examples of fungicidal compounds which may be included in the composition of the invention are (RS)-1-aminopropylphosphonic acid, (RS)-4--(4-chlorophenyl)-2-phenyl-2-(1H-1,2,4-triazol-1-ylmethyl)butyronitrile, (Z)-N-but-2-enyloxymethyl-2-chloro-2',6'-diethylacetanilide, 1-(2-cyano-2--methoxyiminoacetyl)-3-ethyl urea, 3-(2,4-dichlorophenyl)-2-(1H-1,2,4-triazol-1-yl)quinazolin-4(3H)-one, 4-bromo-2-cyano-N,N-dimethyl-6-trifluoromethylbenzimidazole-1-sulphonamide, 5-ethyl-5,8-dihydro-8-oxo(1,3)-dioxol-(4,5-g)quinoline-7-carboxylic acid, α-[N-(3-chloro-2,6-xylyl)-2-methoxyacetamido]-γ-butyrolactone, aldimorph, anilazine, benalaxyl, benomyl, biloxazol, binapacryl, bitertanol, blasticidin S, bromuconazole, bupirimate, buthiobate, captafol, captan, carbendazim, carboxin, chlorbenzthiazone, chloroneb, chlorothalonil, chlorozolinate, copper containing compounds such as copper oxychloride, copper sulphate and Bordeaux mixture, cycloheximide, cymoxanil,

cyproconazole, cyprofuram, di-2-pyridyl disulphide 1,1'-dioxide, dichlofluanid, dichlone, diclobutrazol, diclomezine, dicloran, difenoconazole, dimethamorph, dimethirimol, diniconazole, dinocap, ditalimfos, dithianon, dodemorph, dodine, edifenphos, etaconazole, ethirimol, ethyl (Z)-N-benzyl-N-([methyl(methylthioethylideneamino-oxycarbonyl)amino]thio)-$\beta$-alaninate, etridiazole, fenapanil, fenarimol, fenfuram, fenpiclonil, fenpropidin, fenpropimorph, fentin acetate, fentin hydroxide, flutolanil, flutriafol, flusilazole, folpet, fosetyl-aluminium, fuberidazole, furalaxyl, furconazole-cis, guazatine, hexaconazole, hydroxyisoxazole, imazalil, imibenconazole, iprobenfos, iprodione, isoprothiolane, kasugamycin, mancozeb, maneb, mepanipyrim, mepronil, metalaxyl, methfuroxam, metsulfovax, myclobutanil, neoasozin, nickel dimethyldithiocarbamate, nitrothal-isopropyl, nuarimol, ofurace, organomercury compounds, oxadixyl, oxycarboxin, pefurazoate, penconazole, pencycuron, phenazin oxide, phthalide, polyoxin D, polyram, probenazole, prochloraz, procymidone, propamocarb, propiconazole, propineb, prothiocarb, pyrazophos, pyrifenox, pyroquilon, pyroxyfur, pyrrolnitrin, quinomethionate, quintozene, SSF-109, streptomycin, sulphur, tebuconazole, techlofthalam, tecnazene, tetraconazole, thiabendazole, thicyofen, thiophanate-methyl, thiram, tolclofos-methyl, triacetate salt of 1,1'-iminodi(octamethylene)diguanidine, triadimefon, triadimenol, triazbutyl, tricyclazole, tridemorph, triforine, validamycin A, vinclozolin, zarilamid and zineb. The compounds of general formula (I) can be mixed with soil, peat or other rooting media for the protection of plants against seed-borne, soil-borne or foliar fungal diseases.

Suitable insecticides which may be incorporated in the composition of the invention include buprofezin, carbaryl, carbofuran, carbosulfan, chlorpyrifos, cycloprothrin, demeton-s-methyl, diazinon, dimethoate, ethofenprox, fenitrothion, fenobucarb, fenthion, formothion, isoprocarb, isoxathion, monocrotophos, phenthoate, pirimicarb, propaphos and XMC.

Plant growth regulating compounds are compounds which control weeds or seedhead, formation, or selectively control the growth of less desirable plants (e.g. grasses).

Examples of suitable plant growth regulating compounds for use with the invention compounds are 3,6-dichloropicolinic acid, 1-(4-chlorophenyl)-4,6-di-methyl-2-oxo-1,2-dihydropyridine-3-carboxylic acid, methyl-3,6-dichloroanisate, abscisic acid, asulam, benzoylprop-ethyl, carbetamide, daminozide, difenzoquat, dikegulac, ethephon, fenpentezol, fluoridamid, glyphosate, glyphosine, hydroxybenzonitriles (e.g. bromoxynil), inabenfide, isopyrimol, long chain fatty alcohols and acids, maleic hydrazide, mefluidide, morphactins (e.g. chlorfluoroecol), paclobutrazol, phenoxyacetic acids (e.g. 2,4-D or MCPA), substituted benzoic acid (e.g. triiodobenzoic acid), substituted quaternary ammonium and phosphonium compounds (e.g. chloromequat, chlorphonium or mepiquatchloride), tecnazene, the auxins (e.g. indoleacetic acid, indolebutyric acid, naphthylacetic acid or naphthoxyacetic acid), the cytokinins (e.g. benzimidazole, benzyladenine, benzylaminopurine, diphenylurea or kinetin), the gibberellins (e.g. $GA_3$, $GA_4$ or $GA_7$) and triapenthenol.

The following Examples illustrate the invention.

Throughout the Examples the term 'ether' refers to diethyl ether, magnesium sulphate was used to dry solutions and solutions were concentrated under reduced pressure. Where shown, infrared and NMR data are selective; no attempt is made to list every absorption in all cases. The following abbreviations are used throughout:

| | |
|---|---|
| s = singlet | d = doublet |
| dd = doublet of doublets | t = triplet |
| m = multiplet | b = broad |
| mp = melting point | DMF = N,N-dimethylformamide |
| NMR = nuclear magnetic resonance | |

## EXAMPLE 1

This example illustrates the preparation of 4-trimethylacetamido-2-iodo-N,N-dimethylbenzamide (Compound No. 1 of Table I).

### Step 1

Preparation of 4-nitro-2-trifluoroacetamidobenzoic acid.

To a stirred suspension of 4-nitroanthranilic acid (10g) in dry ether (600ml) was added sodium carbonate (80g). The suspension was cooled to 2°C and trifluoroacetic anhydride (85ml) was added

dropwise. The resulting yellow-green mixture was warmed to room temperature and stirred for 5 hours before being poured into chloroform. Excess anhydride was destroyed with ice and the organic layer was washed with water, dried, filtered, and concentrated under reduced pressure to give an orange solid. Chromatography on silica, eluting with ethyl acetate/hexane/acetic acid (50:60:5) gave 4-nitro-2-trifluoroacetamidobenzoic acid (4g) as a yellow solid; $^1$H NMR ($CDCl_3$, 270 MHz) δ 8.06(1H,d), 8.38(1H,d), 9.47(1H,bs), 13.22(1H,bs) ppm; mp 130 °C (decomposition).

Step 2

Preparation of 4-nitro-2-trifluoroacetamido-N,N-dimethylbenzamide.

To a stirred suspension of 4-nitro-2-trifluoroacetamidobenzoic acid (3.5g) and DMF (3 drops) in dichloromethane (30ml) was slowly added oxalyl chloride (1.1ml). Further dichloromethane (70ml) was added to solubilize the mixture which was stirred for 1.5 hours before being slowly added to dimethylamine (40% w/w aq.; 14ml) and 4-dimethylaminopyridine (catalytic amount) at 5 °C. The resulting bright-yellow mixture was stirred for 1.5 hours at 5 °C, then washed with dilute aqueous HCl, saturated aqueous sodium bicarbonate, and water. The organic layer was dried, filtered, and concentrated under reduced pressure to give a yellow oil (3g) which solidified on treatment with ethyl acetate/hexane (1:1). Trituration with a small amount of ethyl acetate/hexane (1:1) gave 4-nitro-2-trifluoroacetamido-N,N-dimethylbenzamide as a pale yellow solid (1.85g); $^1$H NMR ($CDCl_3$, 270 MHz) δ: 3.15(6H,bs), 7.55(1H,d), 8.08(1H,dd), 9.18(1H,s), 10.55-(1H,s) ppm; mp 139.5-140.5 °C.

Step 3

Preparation of 4-amino-2-trifluoroacetamido-N,N-dimethylbenzamide.

Palladium on charcoal (10% on charcoal; catalytic amount) was flushed well with argon. 4-nitro-2-trifluoroacetamido-N,N-dimethylbenzamide (1.3g) in methanol (4.5ml) was added and the reaction vessel flushed three times with hydrogen and kept under hydrogen for 3 hours. The mixture was twice filtered through hyflo and through filter paper to remove the catalyst. Concentration under reduced pressure gave 4-amino-2-trifluoroacetamido-N,N-dimethylbenzamide as a pale yellow solid (1.09g); $^1$H NMR ($CDCl_3$, 270 MHz) δ: 3.1(6H,s), 4.7(2H,s), 6.45(1H,dd), 7.17(1H,d), 7.68(1H,s), 11.4(1H,s) ppm; mp 170-171 °C.

Step 4

Preparation of 4-trimethylacetamido-2-trifluoroacetamido-N,N-dimethyl-benzamide.

Trimethylacetyl chloride (384μl) was added dropwise to a stirred solution of 4-amino-2-trifluoroacetamido-N,N-dimethylbenzamide (800mg), triethylamine (610μl), and 4-dimethylaminopyridine (catalytic amount) in dichloromethane (40ml). After 18 hours, the reaction mixture was washed with dilute aqueous HCl, saturated aqueous sodium bicarbonate, and water, dried, filtered and concentrated under reduced pressure to give an orange oil which crystallised on trituration with hexane. Chromatography on silica, eluting with hexane/ethyl acetate (1:1) gave 4-trimethylacetamido--2-trifluoroacetamido-N,N-dimethylbenzamide as white crystals; $^1$H NMR ($CDCl_3$, 270MHz) δ: 1.3(9H,s), 3. 1(6H,s), 7.45(1H,d), 7.68(1H,s), 7.96-(1H,dd), 8.1(1H,s) ppm; mp 190-191 °C.

Step 5

Preparation of 4-trimethylacetamido-2-amino-N,N-dimethylbenzamide.

To a stirred solution of potassium carbonate in aqueous methanol (40ml; 7%; 2:5 v/v) was added 4-trimethylacetamido-2-trifluoroacetamido--N,N-dimethylbenzamide (360mg). After 32 hours, the reaction mixture was poured into water and the product extracted with ether (2x). The combined organic extracts were dried, filtered, and concentrated under reduced pressure to give 4-trimethylacetamido-2-amino-N,N-dimethylbenzamide as a yellow solid (164mg); $^1$H NMR ($CDCl_3$, 270MHz) δ: 1.3(9H,s), 3.05(6H,s), 4.65-(1H,bs), 6.85(1H,dd), 7.03(1H,d), 7.22(1H,s), 8.10(1H,s) ppm; mp 175-175.5 °C.

Step 6

Preparation of 4-trimethylacetamido-2-iodo-N,N-dimethylbenzamide.

A solution of sodium nitrite (220mg) in water (1ml) was added to stirred solution of 4-trimethylacetamido-2-amino-N,N-dimethylbenzamide (740mg) in concentrated aqueous HCl (5ml) keeping

the temperature below 0°C. After 15 minutes kept at -2°C, the reaction mixture was added to aqueous potassium iodide (560mg in 2ml) and stirred overnight. The mixture was dissolved in ethyl acetate and washed with aqueous sodium hydroxide (10%), water, aqueous sodium sulfate (5%), and water. The organic extract was dried, filtered, and concentrated under reduced pressure to give a yellow solid (610mg). Chromatography on silica, eluting with ethyl acetate gave 4-trimethylacetamido-2-iodo-N,N-dimethylbenzamide as a pale yellow solid (200mg); $^1$H NMR ($CDCl_3$,270 MHz) $\delta$: 1.3(9H,s), 2.85(3H,s), 3.12(3H,s), 7.02-(1H,d), 7.47(1H,s), 7.9(1H,s), 8.0(1H,s) ppm; mp 228.5-230.5°C.

The following are examples of compositions suitable for agricultural and horticultural purposes which can be formulated from the compounds of the invention. Such compositions form another aspect of the invention. Percentages are by weight.

EXAMPLE 2

This Example illustrates the preparation of 4-(2-fluoro-2-methylpropanamido)-2-iodo-N,N-dimethylbenzamide (Compound No. 5 of Table I).

Step 1

Preparation of 2-iodo-4-nitrobenzoic acid.

To a stirred solution of 4-nitroanthranilic acid (1.82g) in concentrated aqueous HCl (30ml) cooled to -5°C was added sodium nitrite (760mg) in water (3ml) keeping the temperature below 0°C. The mixture was cooled to -5°C and, after 15 minutes, it was added to a solution of potassium iodide (2g) in water (10ml) and stirred for 20 hours. The mixture was diluted with ethyl acetate, and the organic layer washed with 10% aqueous sodium thiosulphate, dried ($MgSO_4$) and concentrated under reduced pressure to give a yellow solid (1.83g) which was used without further purification in Step 2; $^1$H NMR (270MHz; $CDCl_3$) $\delta$ 8.12-(1H,d), 8.3(1H,dd), 8.88(1H,d) ppm.

Step 2

Preparation of 2-iodo-4-nitro-N,N-dimethylbenzamide.

Oxalyl chloride (6.7ml) was added dropwise to a stirred suspension of 2-iodo-4-nitrobenzoic acid (23g) in dichlormethane (200ml) under a nitrogen atmosphere. After 2.5 hours, all the solid had dissolved. The solution was added to dimethylamine (40% aq; 41ml) and the resulting mixture was stirred for 4.5 hours. The dichloromethane solution was washed with dilute aqueous HCl, saturated aqueous sodium bicarbonate and water, dried ($MgSO_4$) and concentrated under reduced pressure to give the product as a dark yellow solid (22.09g); $^1$H NMR (270MHz; $CDCl_3$) $\delta$ 2.85(3H,s), 3.18(3H,s), 7.39(1H,d), 8.28(1H,dd), 8.69(1H,dd) ppm.

Step 3

Preparation of 4-amino-2-iodo-N,N-dimethylbenzamide.

2-iodo-4-nitro-N,N-dimethylbenzamide (1.28g) was added portionwise to a solution of tin-II-chloride (2.3g) in concentrated aqueous HCl at 0°C. The mixture was warmed to room temperature after 20 minutes. After 1 hour, the mixture was poured into water, basified with aqueous sodium hydroxide and extracted twice with dichloromethane. The combined organic extracts were dried ($MgSO_4$) and concentrated under reduced pressure to give the product as a yellow solid (950mg); $^1$H NMR (270MHz; $CDCl_3$) $\delta$ 2.88(3H,s), 3.10(3H,s), 3.79(2H,s), 6.65(1H,dd), 6.97(1H,d), 7.12(1H,dd) ppm.

Step 4

Preparation of 4-(2-fluoro-2-methylpropanamido)-2-iodo-N,N-dimethylbenzamide.

A solution of 2-fluoro-2-methylpropanoyl chloride was prepared by treatment of 2-fluoro-2-methylpropanoic acid (426mg) and DMF (2 drops) in dichloromethane (5ml) with oxalyl chloride (350µl) and stirring for 1.5 hours. The solution was added dropwise to a stirred solution of 4-amino-2-iodo-N,N-dimethylbenzamide (950mg), triethylamine (0.7ml) and N,N-dimethylaminopyridine (catalytic amount) in dichloromethane (45ml) and the mixture stirred for 2 hours. The solution was washed with dilute aqueous HCl, saturated aqueous sodium bicarbonate and water, dried ($MgSO_4$) and concentrated under reduced pressure to give a yellow foam (985mg). Chromatography on silica, eluting with ethyl acetate, gave the product as a cream solid (850mg); mp 165.5-168°C.

EXAMPLE 3

This Example illustrates the preparation of 4-(2-fluoro-2-methylpropanamido)-2-ethynyl-N,N-dimethylbenzamide (Compound No. 33 of Table I).

Step 1

Preparation of 4-nitro-2-(2-trimethylsilylethynyl)-N,N-dimethylbenzamide.

A mixture of 2-iodo-4-nitro-N,N-dimethylbenzamide (from Example 2, Step 2) (1.92g), trimethylsilylethyne (1ml), bis(triphenylphosphine)palladium-II-chloride (192mg), copper-I-iodide (192mg) and triethylamine (1.3ml) in acetonitrile (40ml) was stirred under a nitrogen atmosphere for 2 hours. The reaction mixture was diluted with ether, washed with dilute aquesous HCl, water and aqueous ammonia, dried ($MgSO_4$) and concentrated under reduced pressure to give the product as a yellow solid; $^1H$ NMR (270MHz; $CDCl_3$) $\delta$ (inter alia) 2.90(3H,s), 3.15(3H,s), 7.50(1H,d), 8.20(1H,dd), 8.32(1H,d) ppm.

Step 2

Preparation of 4-nitro-2-ethynyl-N,N-dimethylbenzamide.

4-Nitro-2-(2-trimethylsilylethynyl)-N,N-dimethylbenzamide (1.58g) was dissolved in methanol (30ml) and potasium carbonate (200mg) was added in one portion. After 1 hour, the solids were removed by filtration, and the filtrate concentrated under reduced pressure and redissolved in dichloromethane. The solution was washed with water, dried ($MgSO_4$) and concentrated under reduced pressure to give a dark yellow solid which was used in Step 3 without further purification; $^1H$ NMR (270MHz; $CDCl_3$) $\delta$ 2.90(3H,s), 3.35(1H,s), 3.18(3H,s), 7.50(1H,d), 8.25(1H,dd), 8.39(1H,d) ppm.

Step 3

Preparation of 4-amino-2-ethynyl-N,N-dimethylbenzamide.

4-Nitro-2-ethynyl-N,N-dimethylbenzamide (960mg) was added portionwise to a solution of tin-II-chloride (2.5g) in concentrated aqueous HCl (30ml) at 0°C. After 1 hour, the mixture was poured into water and basified with aqueous sodium hydroxide. The aqueous layer was extracted with dichloromethane, dried ($MgSO_4$) and concentrated under reduced pressure to give a yellow oil (760mg) which was used without further purification in Step 4; $^1H$ NMR (270MHz; $CDCl_3$) $\delta$ 2.90(3H,s), 3.10(3H,s), 3.10(1H,s), 3.80(2H,bs), 6.68(1H,dd), 6.79(1H,d), 7.10(1H,d) ppm.

Step 4

Preparation of 4-(2-fluoro-2-methylpropanamido)-2-ethynyl-N,N-dimethylbenzamide.

A solution of 2-fluoro-2-methylpropanoyl chloride was prepared by treatment of 2-fluoro-2-methylpropanoic acid (263mg) and DMF (3 drops) in dichloromethane (10ml) with oxalyl chloride (215µl) and stirring for 1.5 hours. The solution was added dropwise to a stirred solution of 4-amino-2-ethynyl-N,N-dimethylbenzamide (360mg), triethylamine (0.6ml) and N,N-dimethylaminopyridine (catalytic amount) in dichloromethane (25ml) and the mixture stirred for 1.5 hours. The solution was washed with dilute aqueous HCl, saturated aqueous sodium bicarbonate and water, dried ($MgSO_4$) and concentrated under reduced pressure to give a brown oil (590mg) which crystallised on standing. Chromatography on silica, eluting with ethyl acetate, gave the pure product as a yellow oil which cryatallised on standing (317mg); mp 110°C (decomp.)

EXAMPLE 4

This Example illustrates the preparation of 4-(2-fluoro-2-methylpropanamido)-2-ethenyl-N,N-dimethylbenzamide (Compound No. 34 of Table I).

Step 1

Preparation of 4-nitro-2-ethenyl-N,N-dimethylbenzamide.

A stirred mixture of 2-iodo-4-nitro-N,N-dimethylbenzamide (from Example 2, Step 2) (1g), bis-(triphenylphosphine)palladium-II-chloride (80mg) and vinyltributyltin (915µl) in dry DMF (20ml) was heated

at 70 °C under a nitrogen atmosphere for 12 hours. The cooled mixture was added to aqueous potassium fluoride (10%) and ether, and the resulting mixture stirred for 1 hour before filtration through hyflo. The ether layer was separated and the aqueous layer further extracted with ether. The combined ether extracts were washed with brine, dried ($MgSO_4$) and concentrated under reduced pressure to give an orange oil which was triturated with hexane. The residue was used without further purification in Step 2; $^1H$ NMR (270MHz; $CDCl_3$) δ 2.50(3H,s), 3.16(3H,s), 5.52(1H,d), 5.95(1H,d), 6.73(1H,dd), 7.40(1H,d), 8.15(1H,dd), 8.44-(1H,d) ppm.

Step 2

Preparation of 4-amino-2-ethenyl-N,N-dimethylbenzamide.

4-Nitro-2-ethenyl-N,N-dimethylbenzamide (690mg) was added portionwise to a soltuion of tin-II-chloride (1.8g) in concentrated aqueous HCl (30ml) at 0 °C. After 1 hour, the mixture was poured into water and basified with aqueous sodium hydroxide. The insoluble materials were removed by filtration. The aqueous layer was extracted with dichloromethane, dried ($MgSO_4$) and concentrated under reduced pressure to give a yellow oil (457mg) which was used without further purification in Step 3; $^1H$ NMR (270MHz; $CDCl_3$) δ 2.80-(3H,s), 3.11(3H,s), 3.78(2H,s), 5.26(1H,d), 5.67(1H,d), 6.61(1H,dd), 6.65(1H,dd), 6.85(1H,d), 7.03(1H,d) ppm.

Step 3

Preparation of 4-(2-fluoro-2-methylpropanamido)-2-ethenyl-N,N-dimethylbenzamide.

A solution of 2-fluoro-2-methylpropanoyl chloride was prepared by treatment of 2-fluoro-2-methylpropanoic acid (300mg) and DMF (2 drops) in dichloromethane (5ml) with oxalyl chloride (250µl) and stirring for 1 hour. The solution was added dropwise to a stirred solution of 4-amino-2-ethenyl-N,N-dimethylbenzamide (450mg), triethylamine (0.5ml) and N,N-dimethylaminopyridine (catalytic amount) in dichloromethane (20ml) and the mixture stirred for 1 hour. The solution was washed with dilute aqueous HCl, saturated aqueous sodium bicarbonate and water, dried ($MgSO_4$) and concentrated under reduced pressure to give a yellow oil which crystallised on standing. Recrystallisation from ethyl acetate/hexane gave the product as yellow crystals (327mg); mp 164-165 °C.

EXAMPLE 5

An emulsifiable concentrate is made up by mixing and stirring the ingredients until all are dissolved.

| | |
|---|---|
| Compound No. 1 of Table I | 10% |
| Benzyl alcohol | 30% |
| Calcium dodecylbenzenesulphonate | 5% |
| Nonylphenolethoxylate (13 mole ethylene oxide) | 10% |
| Alkyl benzenes | 45% |

EXAMPLE 6

The active ingredient is dissolved in methylene dichloride and the resultant liquid sprayed on to the granules of attapulgite clay. The solvent is then allowed to evaporate to produce a granular composition.

| | |
|---|---|
| Compound No. 1 of Table I | 5% |
| Attapulgite granules | 95% |

EXAMPLE 7

A composition suitable for use as a seed dressing is prepared by grinding and mixing the three ingredients.

| | |
|---|---|
| Compound No. 1 of Table I | 50% |
| Mineral oil | 2% |
| China clay | 48% |

EXAMPLE 8

A dustable powder is prepared by grinding and mixing the active ingredient with talc.

| | |
|---|---|
| Compound No. 1 of Table I | 5% |
| Talc | 95% |

EXAMPLE 9

A suspension concentrate is prepared by ball milling the ingredients to form an aqueous suspension of the ground mixture with water.

| | |
|---|---|
| Compound No. 1 of Table I | 40% |
| Sodium lignosulphonate | 10% |
| Bentonite clay | 1% |
| Water | 49% |

This formulation can be used as a spray by diluting into water or applied directly to seed.

EXAMPLE 10

A wettable powder formulation is made by mixing together and grinding the ingredients until all are thoroughly mixed.

| | |
|---|---|
| Compound No. 1 of Table I | 25% |
| Sodium lauryl sulphate | 2% |
| Sodium lignosulphonate | 5% |
| Silica | 25% |
| China clay | 43% |

EXAMPLE 11

The compounds were tested against a variety of foliar fungal diseases of plants. The technique employed was as follows.

The plants were grown in John Innes Potting Compost (No. 1 or 2) in 4cm diameter minipots. The test compounds were formulated either by bead milling with aqueous Dispersol T or as a solution in acetone or acetone/ethanol which was diluted to the required concentration immediately before use. For the foliage diseases, the formulations (100 ppm active ingredient) were sprayed onto the foliage and applied to the roots of the plants in the soil. The sprays were applied to maximum retention and the root drenches to a final concentration equivalent to approximately 40 ppm a.i. in dry soil. Tween 20, to give a final concentration of 0.05%, was added when the sprays were applied to cereals. For most of the tests the compound was applied to the soil (roots) and to the foliage (by spraying) one or two days before the plant was inoculated with the disease. An exception was the test on Erysiphe graminis

in which the plants were inoculated 24 hours before treatment. Foliar pathogens were applied by spray as spore suspensions onto the leaves of test plants. After inoculation, the plants were put into an appropriate environment to allow infection to proceed and then incubated until the disease was ready for assessment. The period between inoculation and assessment varied from four to fourteen days according to

the disease and environment.

The disease control was recorded by the following grading:

4 = no disease
3 = trace-5% of disease on untreated plants
2 = 6-25% of disease on untreated plants
1 = 26-59% of disease on untreated plants
0 = 60-100% of disease on untreated plants

The results are shown in Table II.

TABLE II

| Compound No. | Pr | Egt | Sn | Po | Vi | Pv | Pil |
|---|---|---|---|---|---|---|---|
| 1 | 0 | 0 | 0 | 0 | 0 | 4 | 4 |
| 5 | 0[a] | 0[a] | 0[a] | 0[a] | 0[a] | 4[a] | 4[a] |
| 13 | 0 | 3 | 3 | 3 | 4 | 3 | 3 |
| 19 | 0[a] | 0[a] | 0[a] | 0[a] | 3[a] | 0[a] | 0[a] |
| 23 | 0[a] | 0[a] | 0[a] | 0[a] | 0[a] | 4[a] | 0[a] |
| 29 | - | 1 | 1 | - | 0 | 0 | 0 |
| 33 | - | 0 | - | 0 | 0 | 4 | 4 |
| 34 | - | 0 | - | 0 | 4 | 4 | 4 |
| 35 | - | 0 | - | 0 | 0 | 4 | 4 |

a = root drench application only @ 25 ppm.
b = root drench application only @ 100 ppm.

Key to Diseases
Pr Puccinia recondita
Egt Erysiphe graminis tritici
Sn Septoria nodorum
Po Pyricularia oryzae
Vi Venturia inaequalis
Pv Plasmopara viticola
Pil Phytophthora infestans lycopersici

CHEMICAL FORMULAE
(in description)

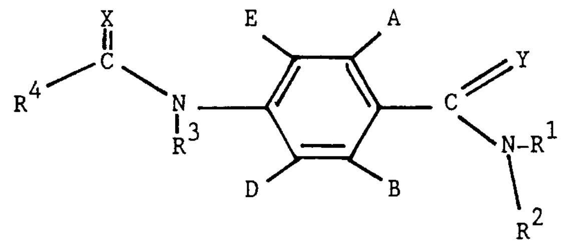

(I)

Scheme 1

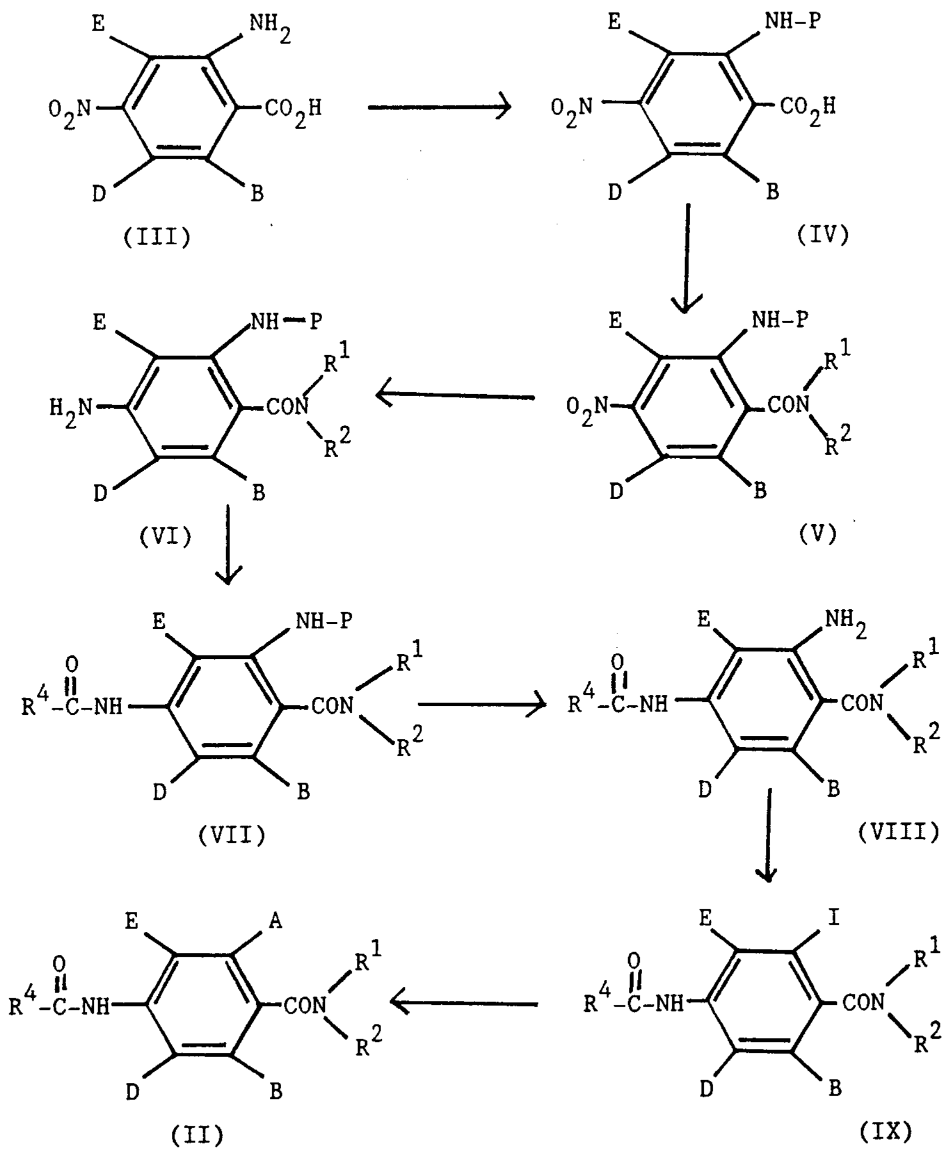

(III) (IV) (V) (VI) (VII) (VIII) (IX) (II)

Scheme 2

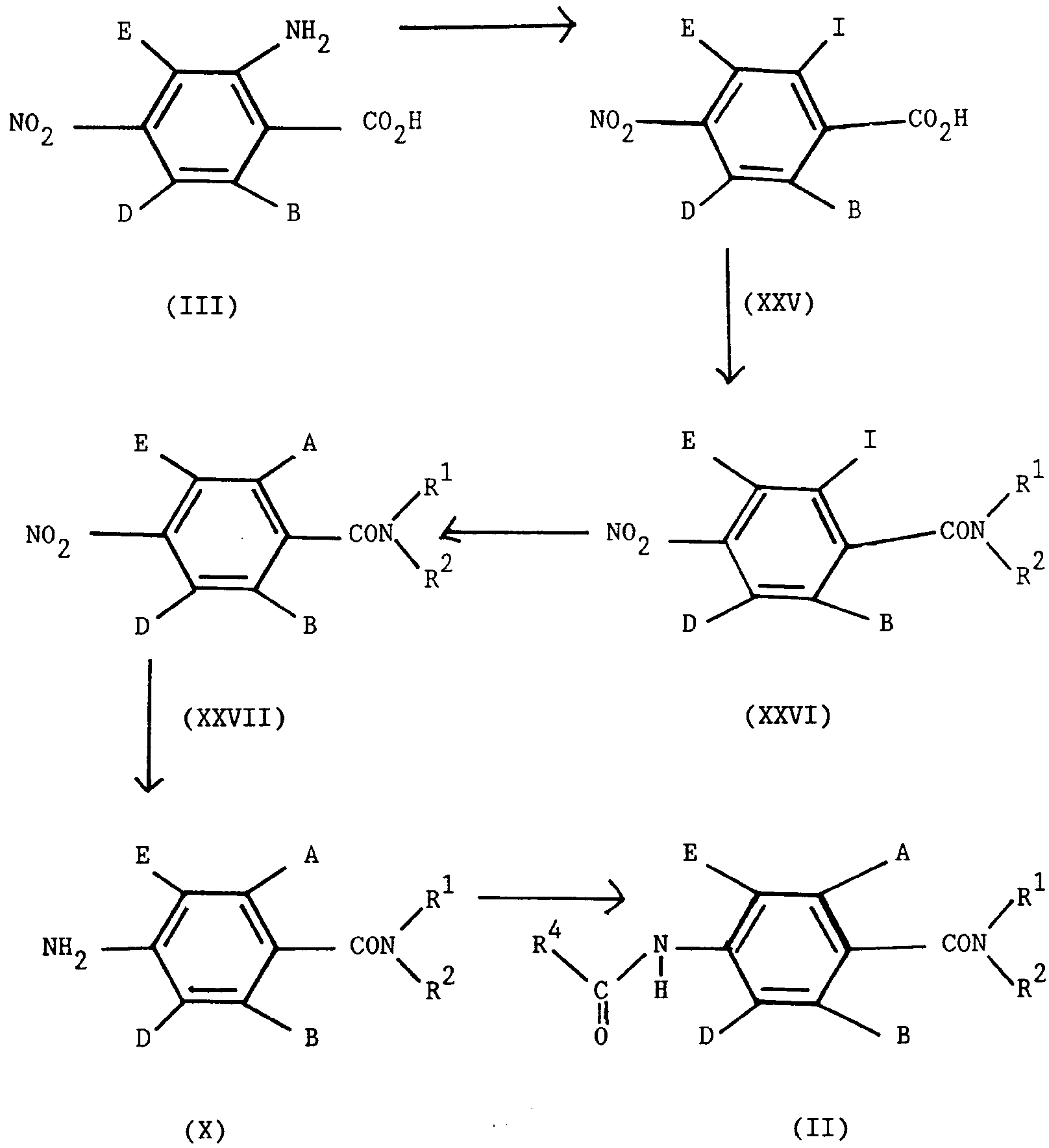

(III) (XXV)

(XXVII) (XXVI)

(X) (II)

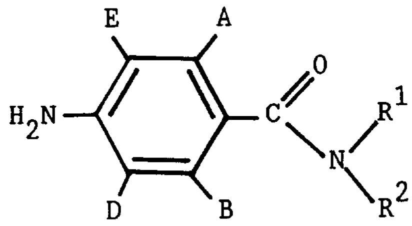

(X)

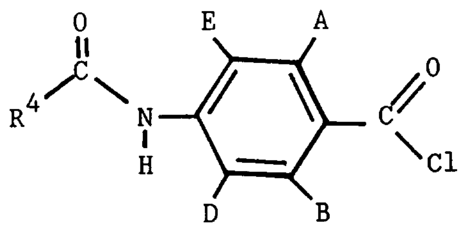

(XI)

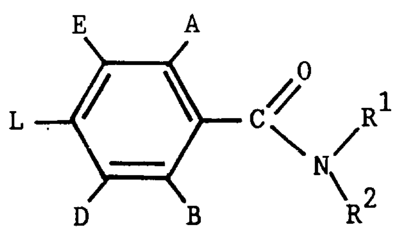

(XII)

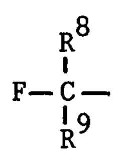

(XV)

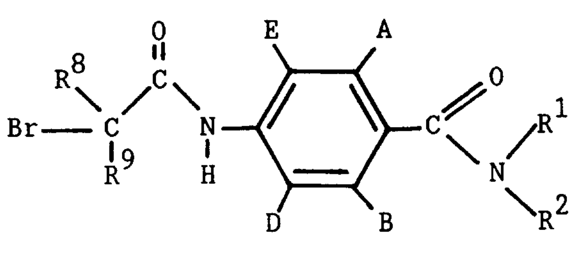

(XVI)

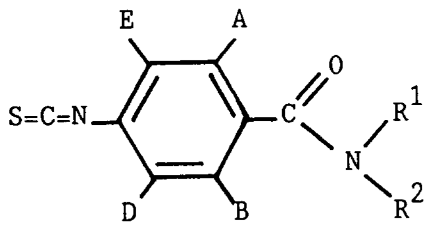

(XXI)

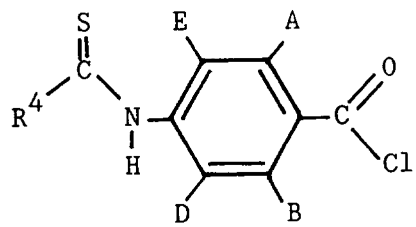

(XXII)

## Claims

**Claims for the following Contracting States : AT, BE, CH, DE, DK, FR, GB, GR, IT, LI, LU, NL, SE**

1. A compound of the formula (I):

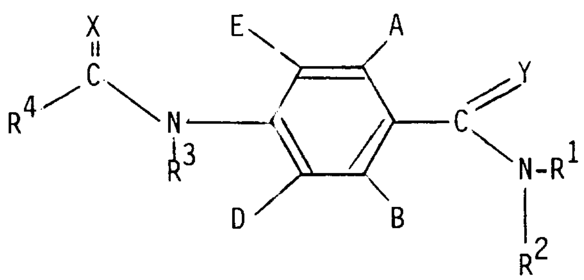

(I)

in which A and B are independently H, iodo, nitro, cyano, $C_{1-4}$ alkoxycarbonyl, $C_{1-4}$ alkoxy($C_{1-4}$)alkyl, $C_{1-4}$ alkylthio($C_{1-4}$)alkyl, formyl, $C_{1-4}$ alkylthio, $C_{1-4}$alkylcarbonyl, -$CR^5$ = $NOR^6$ (where $R^5$ and $R^6$ are independently H or $C_{1-4}$ alkyl), $C_{2-4}$ alkenyl or $C_{2-4}$ alkynyl, provided that A and B are not both H; D and E are independently H or fluoro; $R^1$ is $C_{1-4}$ alkyl or $C_{1-4}$ alkoxy; $R^2$ is $C_{1-4}$ alkyl or $C_{1-4}$ alkoxy, or $R^1$ and $R^2$ together with the nitrogen atom to which they are attached join to form a morpholine, piperidine, pyrrolidine or azetidine ring which is optionally substituted with $C_{1-4}$ alkyl; $R^3$ is H; $R^4$ is R-$(CH_3)_2C$, where R is halo, $C_{1-4}$ alkyl or $C_{1-4}$ alkoxy; and X and Y are independently oxygen or sulphur.

2. A compound according to claim 1 in which $R^4$ is $F(CH_3)_2C$.

3. A compound according to claim 1 in which A is iodo, cyano, nitro, $C_{1-4}$ alkoxycarbonyl, $C_{1-4}$ alkylcarbonyl, $C_{1-4}$ alkoxy($C_{1-4}$)alkyl, $C_{1-4}$ alkylthio, formyl, $C_{1-4}$ alkylthio($C_{1-4}$)alkyl or -CH = $NOR^6$ -(where $R^6$ is $C_{1-4}$ alkyl); $R^1$ and $R^2$ are alkyl; $R^3$, B, D and E are H; $R^4$ is $C_{2-6}$ alkyl optionally substituted with halogen or $C_{1-4}$ alkoxy; and X and Y are both oxygen.

4. A process for preparing a compound according to claim 1 in which A is iodo, $R^3$ is H and X and Y are both oxygen, which comprises treating a compound of formula (VIII):

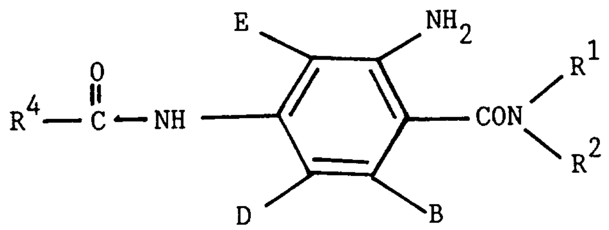

(VIII)

with nitrous acid and an iodide source; wherein B, D, E, $R^1$, $R^2$ and $R^4$ have the meanings given in claim 1.

**5.** A process for preparing a compound according to claim 1 in which A is cyano or $C_{1-4}$ alkylthio, $R^3$ is H and X and Y are both oxygen, which comprises reacting a compound of formula (IX):

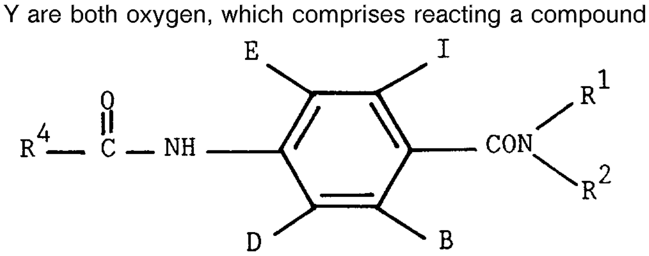

(IX)

with a transition metal salt of a cyanide or $C_{1-4}$ alkanethiolate in a suitable solvent; wherein B, D, E, $R^1$, $R^2$ and $R^4$ have the meanings given in claim 1.

**6.** A process for preparing a compound according to claim 1,

a) when X and Y are both oxygen and $R^3$ is H,

i) by reacting a compound of general formula (X):

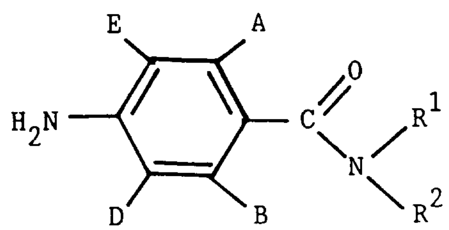

(X)

with an acid chloride $R^4COCl$ in a suitable organic solvent in the presence of a base;

ii) by reacting a compound of general formula (XI):

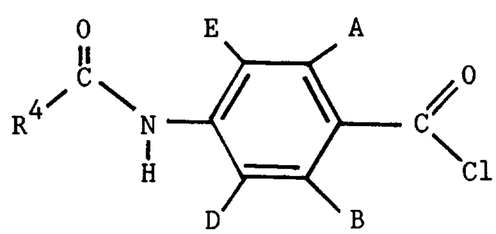

(XI)

with an amine $R^1R^2NH$ in a suitable organic solvent in the presence of a base or excess $R^1R^2NH$; or

iii) by reacting a compound of general formula (XII):

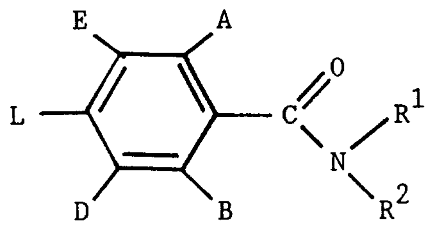

(XII)

with a compound of general formula $R^4$-CO-$NH_2$ and a base;

b) when X and Y are both oxygen, $R^3$ is H and $R^4$ is the group (XV):

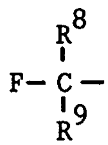

(XV)

i) by treating a compound of general formula (XVI):

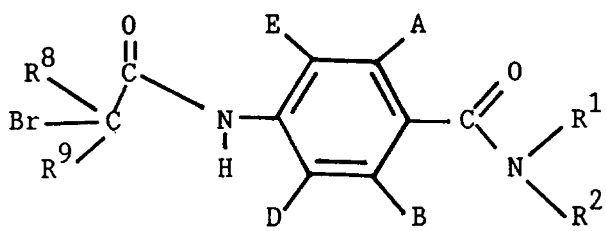

(XVI)

with a fluoride transfer reagent in a suitable solvent;

c) when X is oxygen or sulphur, Y is sulphur and $R^3$ is H,

i) by treating a compound of general formula (II):

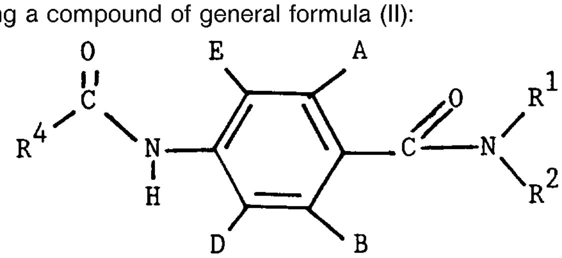

(II)

with a thionation reagent in a suitable solvent to form either a compound of general formula (I) in which X is oxygen, Y is sulphur and $R^3$ is hydrogen or a mixture of a compound of general formula (I) in which X is oxygen, Y is sulphur and $R^3$ is hydrogen and a compound of general formula (I) in which X and Y are both sulphur and $R^3$ is hydrogen;

d) when X is sulphur, Y is oxygen and $R^3$ is H,

i) by reacting an isothiocyanate of general formula (XXI):

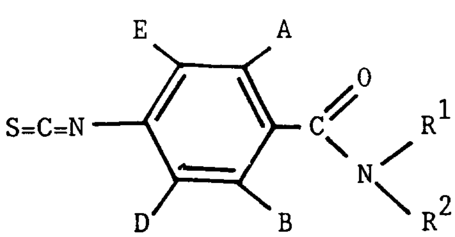

(XXI)

with an organometallic reagent $R^4Li$ or $R^4Mg$-hal in a suitable solvent at a temperature between -78 °C and +25 °C; or

ii) by reacting an acid chloride (XXII):

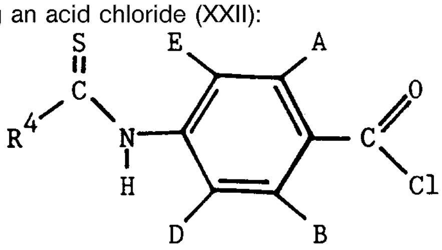

(XXII)

with an amine $R^1R^2NH$ in the presence of a base;

wherein A, B, D, E, $R^1$, $R^2$ and $R^4$ (except where otherwise stated) have the meanings given in claim 1, $R^8$ and $R^9$ are both methyl, hal is halogen and L is a leaving group.

**7.** A fungicidal composition comprising a fungicidally effective amount of a compound according to claim 1 and a fungicidally acceptable carrier or diluent therefor.

**8.** A method of combating fungi which comprises applying to plants, to the seeds of plants or to the locus of the plants or seeds, a compound according to claim 1 or a composition according to claim 7.

**Claims for the following Contracting State : ES**

**1.** A fungicidal composition comprising a compound of the formula (I):

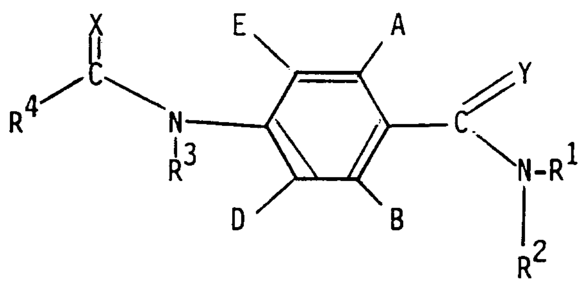

(I)

in which A and B are independently H, iodo, nitro, cyano, $C_{1-4}$ alkoxycarbonyl, $C_{1-4}$ alkoxy($C_{1-4}$)alkyl, $C_{1-4}$ alkylthio($C_{1-4}$)alkyl, formyl, $C_{1-4}$ alkylthio, $C_{1-4}$alkylcarbonyl, $-CR^5=NOR^6$ (where $R^5$ and $R^6$ are independently H or $C_{1-4}$ alkyl), $C_{2-4}$ alkenyl or $C_{2-4}$ alkynyl, provided that A and B are not both H; D and E are independently H or fluoro; $R^1$ is $C_{1-4}$ alkyl or $C_{1-4}$ alkoxy; $R^2$ is $C_{1-4}$ alkyl or $C_{1-4}$ alkoxy, or $R^1$ and $R^2$ together with the nitrogen atom to which they are attached join to form a morpholine, piperidine, pyrrolidine or azetidine ring which is optionally substituted with $C_{1-4}$ alkyl; $R^3$ is H; $R^4$ is R-$(CH_3)_2C$, where R is halo, $C_{1-4}$ alkyl or $C_{1-4}$ alkoxy; and X and Y are independently oxygen or sulphur.

**2.** A composition according to claim 1 in which $R^4$ is $F(CH_3)_2C$.

**3.** A composition according to claim 1 in which A is iodo, cyano, nitro, $C_{1-4}$ alkoxycarbonyl $C_{1-4}$ alkylcarbonyl, $C_{1-4}$ alkoxy($C_{1-4}$)alkyl, $C_{1-4}$ alkylthio, formyl, $C_{1-4}$ alkylthio($C_{1-4}$)alkyl or $-CH=NOR^6$ - (where $R^6$ is $C_{1-4}$ alkyl); $R^1$ and $R^2$ are alkyl; $R^3$ , B, D and E are H; $R^4$ is $C_{2-6}$ alkyl optionally substituted with halogen or $C_{1-4}$ alkoxy; and X and Y are both oxygen.

**4.** A process for preparing a compound of formula (I):

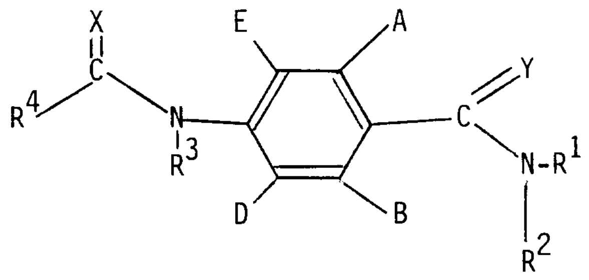

(I)

in which A and B are independently H, iodo, nitro, cyano, $C_{1-4}$ alkoxycarbonyl, $C_{1-4}$ alkoxy($C_{1-4}$)alkyl $C_{1-4}$ alkylthio($C_{1-4}$)alkyl, formyl, $C_{1-4}$ alkylthio, $C_{1-4}$alkylcarbonyl, $-CR^5=NOR^6$ (where $R^5$ and $R^6$ are independently H or $C_{1-4}$ alkyl), $C_{2-4}$ alkenyl or $C_{2-4}$ alkynyl, provided that A and B are not both H; D and E are independently H or fluoro; $R^1$ is $C_{1-4}$ alkyl or $C_{1-4}$ alkoxy; $R^2$ is $C_{1-4}$ alkyl or $C_{1-4}$ alkoxy, or $R^1$ and $R^2$ together with the nitrogen atom to which they are attached join to form a morpholine, piperidine, pyrrolidine or azetidine ring which is optionally substituted with $C_{1-4}$ alkyl; $R^3$ is H; $R^4$ is $R-(CH_3)_2C$, where R is halo, $C_{1-4}$ alkyl or $C_{1-4}$ alkoxy; and X and Y are independently oxygen or sulphur; the process comprising:

(a) for a compound in which A is iodo, $R^3$ is H and X and Y are both oxygen, treating a compound of formula (VIII):

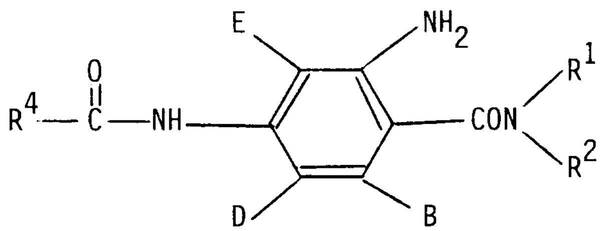

(VIII)

with nitrous acid and an iodide source;

(b) for a compound in which A is cyano or $C_{1-4}$ alkylthio, $R^3$ is H and X and Y are both oxygen, reacting a compound of formula (IX):

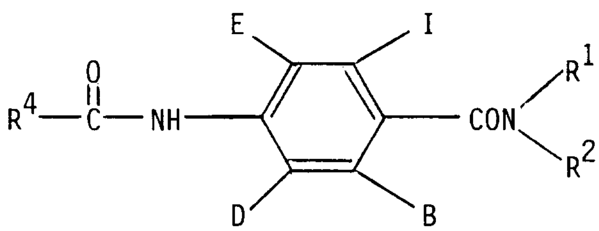

(IX)

with a transition metal salt of a cyanide or $C_{1-4}$ alkanethiolate in a suitable solvent;

(c) when X and Y are both oxygen and $R^3$ is H,

i) by reacting a compound of general formula (X):

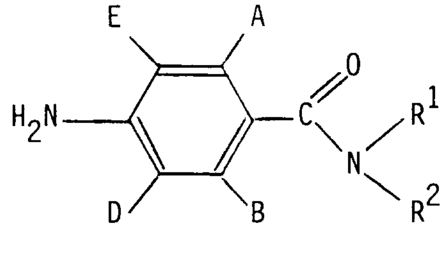

(X)

with an acid chloride $R^4COCl$ in a suitable organic solvent in the presence of a base;

ii) by reacting a compound of general formula (XI):

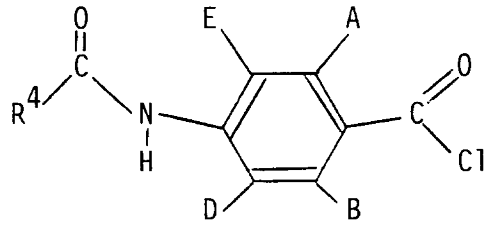

(XI)

with an amine $R^1R^2NH$ in a suitable organic solvent in the presence of a base or excess $R^1R^2NH$; or

iii) by reacting a compound of general formula (XII):

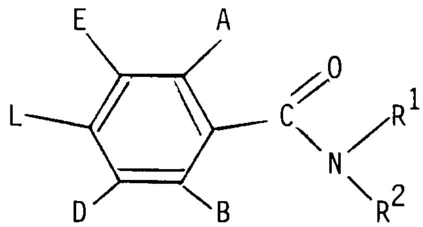

(XII)

with a compound of general formula $R^4$-CO-$NH_2$ and a base;

(d) when X and Y are both oxygen, $R^3$ is H and $R^4$ is the group (XV):

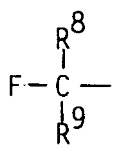

(XV)

i) by treating a compound of general formula (XVI):

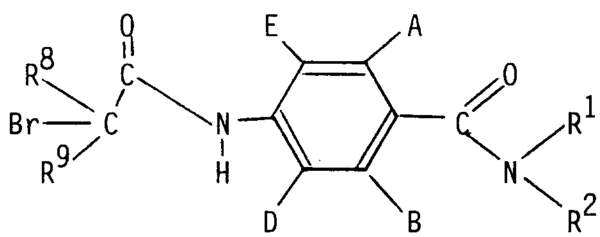

(XVI)

with a fluoride transfer reagent in a suitable solvent;

e) when X is oxygen or sulphur, Y is sulphur and $R^3$ is H, by treating a compound of general formula (II):

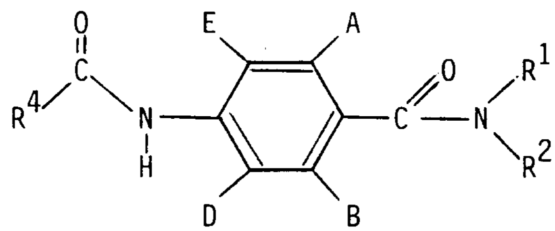

(II)

ith a thionation reagent in a suitable solvent to form either a compound of general formula (I) in which X is oxygen, Y is sulphur and $R^3$ is hydrogen or a mixture of a compound of general formula

(I) in which X is oxygen, Y is sulphur and $R^3$ is hydrogen and a compound of general formula (I) in which X and Y are both sulphur and $R^3$ is hydrogen;

f) when X is sulphur, Y is oxygen and $R^3$ is H,

i) by reacting an isothiocyanate of general formula (XXI):

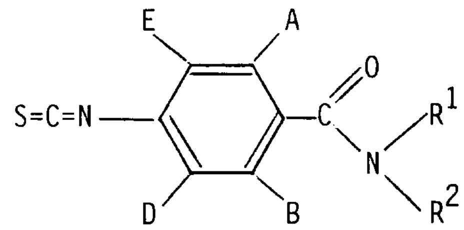

(XXI)

with an organometallic reagent $R^4Li$ or $R^4Mg$-hal in a suitable solvent at a temperature between -78 °C and +25 °C; or

ii) by reacting an acid chloride (XXII) :

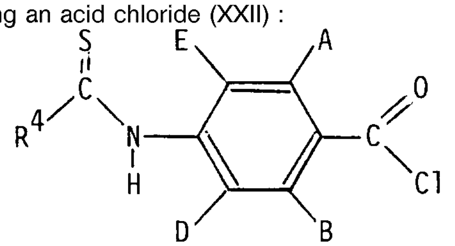

(XXII)

with an amine $R^1R^2NH$ in the presence of a base;

wherein $R^8$ and $R^9$ are both methyl, hal is halogen and L is a leaving group.

**5.** A process according to claim 4 in which $R^4$ is $F(CH_3)_2C$.

**6.** A process according to claim 4 in which A is iodo, cyano, nitro, $C_{1-4}$ alkoxycarbonyl, $C_{1-4}$ alkylcarbonyl, $C_{1-4}$ alkoxy($C_{1-4}$)alkyl, $C_{1-4}$ alkylthio, formyl, $C_{1-4}$ alkylthio($C_{1-4}$)alkyl or -CH=NOR$^6$ (where $R^6$ is $C_{1-4}$ alkyl); $R^1$ and $R^2$ are alkyl; $R^3$ B, D and E are H; $R^4$ is $C_{2-6}$ alkyl optionally substituted with halogen or $C_{1-4}$ alkoxy; and X and Y are both oxygen.

**Patentansprüche**

**Patentansprüche für folgende Vertragsstaaten : AT, BE, CH, DE, DK, FR, GB, GR, IT, LI, LU, NL, SE**

**1.** Verbindung mit der folgenden Formel (I):

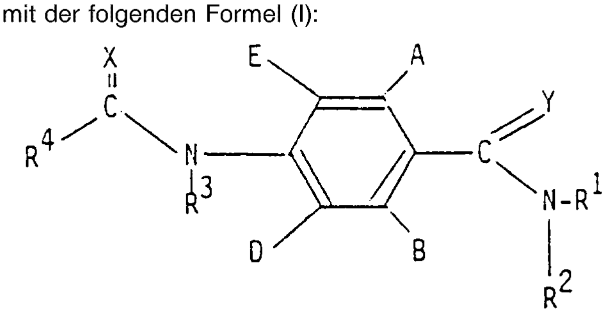

(I)

in der A und B unabhängig voneinander für folgendes stehen: H, Jod, Nitro, Cyano, $C_{1-4}$-Alkoxycarbonyl $C_{1-4}$-Alkoxy($C_{1-4}$)alkyl, $C_{1-4}$-Alkylthio($C_{1-4}$)Alkyl, Formyl, $C_{1-4}$-Alkylthio, $C_{1-4}$-Alkylcarbonyl, -CR$^5$=NOR$^6$ (wobei $R^5$ und $R^6$ unabhängig voneinander für H oder $C_{1-4}$-Alkyl stehen), $C_{2-4}$-Alkenyl oder $C_{2-4}$-Alkinyl, mit der Maßgabe, daß A und B nicht beide für H stehen; D und E unabhängig voneinander für H oder Fluor stehen; $R^1$ für $C_{1-4}$-Alkyl oder $C_{1-4}$-Alkoxy steht; $R^2$ für $C_{1-4}$-Alkyl oder $C_{1-4}$-Alkoxy steht, oder in der $R^1$ und $R^2$ zusammen mit dem Stickstoffatom, an das sie gebunden sind, unter Bildung eines Morpholin-, Piperidin-, Pyrrolidin- oder Azetidin-Rings miteinander verbunden sind, der gegebenenfalls mit $C_{1-4}$-Alkyl substituiert ist; $R^3$ für H steht; $R^4$ für $R(CH_3)_2C$ steht, wobei R

für Halogen, $C_{1-4}$-Alkyl oder $C_{1-4}$-Alkoxy steht; und X und Y unabhängig voneinander für Sauerstoff oder Schwefel stehen.

**2.** Verbindung nach Anspruch 1, wobei der $R^4$ für $F(CH_3)_2C$ steht.

**3.** Verbindung nach Anspruch 1, bei der A für Jod, Cyano, Nitro, $C_{1-4}$-Alkoxycarbonyl, $C_{1-4}$-Alkylcarbonyl, $C_{1-4}$-Alkoxy($C_{1-4}$)alkyl, $C_{1-4}$-Alkylthio, Formyl, $C_{1-4}$-Alkylthio($C_{1-4}$)alkyl oder -CH=NOR$^6$ steht (wobei $R^6$ für $C_{1-4}$-Alkyl steht); $R^1$ und $R^2$ für Alkyl stehen; $R^3$, B, D und E für H stehen; $R^4$ für $C_{2-6}$-Alkyl steht, das gegebenenfalls mit Halogen oder $C_{1-4}$-Alkoxy substituiert ist; und X und Y beide für Sauerstoff stehen.

**4.** Verfahren zur Herstellung einer Verbindung nach Anspruch 1, bei der A für Jod steht, $R^3$ für H steht und X und Y beide für Sauerstoff stehen, bei dem eine Verbindung mit der folgenden Formel (VIII):

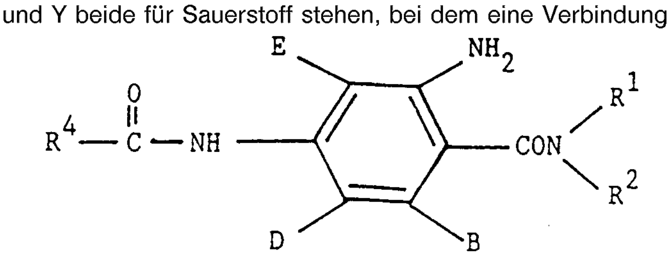

(VIII)

mit salpetriger Säure und einer Jodid-Quelle behandelt wird, wobei B, D, E, $R^1$, $R^2$ und $R^4$ die in Anspruch 1 angegebenen Bedeutungen haben.

**5.** Verfahren zur Herstellung einer Verbindung nach Anspruch 1, bei der A für Cyano, $C_{1-4}$-Alkylthio steht, $R^3$ für H steht und X und Y beide für Sauerstoff stehen, bei dem eine Verbindung mit der folgenden Formel (IX):

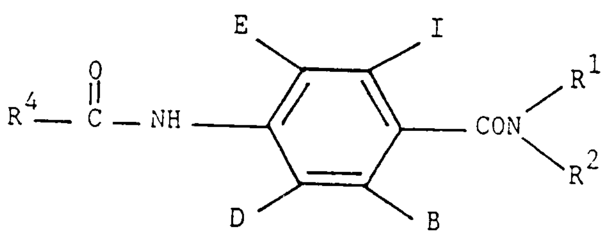

(IX)

mit einem Übergangsmetall-Salz eines Cyanids oder $C_{1-4}$-Alkanthiolats in einem geeigneten Lösungsmittel umgesetzt wird, wobei B, D, E, $R^1$, $R^2$ und $R^4$ die in Anspruch 1 angegebene Bedeutungen haben.

**6.** Verfahren zur Herstellung einer Verbindung nach Anspruch 1, bei dem
a) wenn X und Y beide für Sauerstoff stehen und $R^3$ für H steht,
i) eine Verbindung mit der folgenden allgemeinen Formel (X):

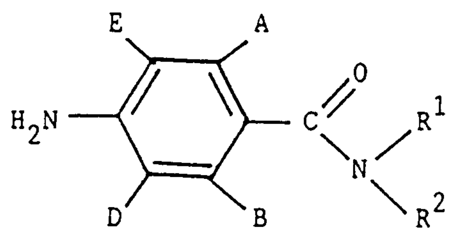

(X)

mit einem Säurechlorid $R^4COCl$ in einem geeigneten organischen Lösungsmittel in der Gegenwart einer Base umgesetzt wird;
ii) eine Verbindung mit der folgenden allgemeinen Formel (XI):

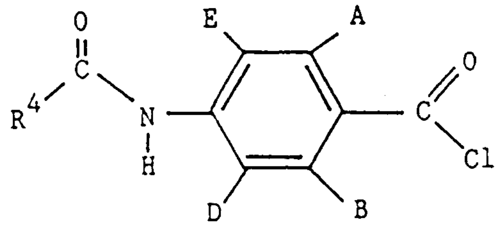

(XI)

mit einem Amin $R^1R^2NH$ in einem geeigneten organischen Lösungsmittel in der Gegenwart einer Base oder eines Überschusses $R^1R^2NH$ umgesetzt wird; oder

iii) eine Verbindung mit der folgenden allgemeinen Formel (XII):

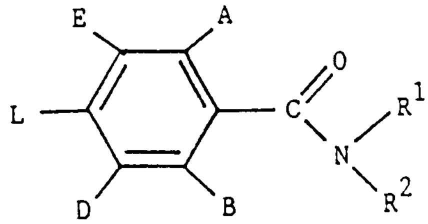

(XII)

mit einer Verbindung mit der allgemeinen Formel $R^4$-CO-$NH_2$ und einer Base umgesetzt wird;

b) wenn X und Y beide für Sauerstoff stehen, $R^3$ für H steht und $R^4$ für die Gruppe (XV) steht:

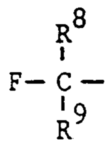

(XV)

i) eine Verbindung mit der folgenden allgemeinen Formel (XVI):

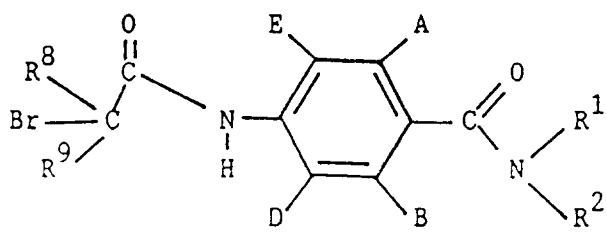

(XVI)

mit einem Fluorid-Übertragungsmittel in einem geeigneten Lösungsmittel behandelt wird;

c) wenn X für Sauerstoff oder Schwefel steht, Y für Schwefel steht und $R^3$ für H steht,

i) eine Verbindung mit der folgenden allgemeinen Formel (II):

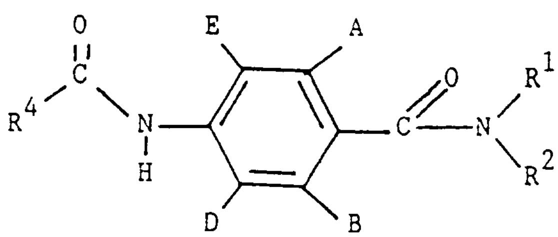

(II)

mit einem Thionierungsreagenz in einem geeigneten Lösungsmittel behandelt wird, und zwar entweder unter Bildung einer Verbindung mit der allgemeinen Formel (I), bei der X für Sauerstoff

steht, Y für Schwefel steht und $R^3$ für Wasserstoff steht, oder eines Gemisches aus einer Verbindung mit der allgemeinen Formel (I), bei der X für Sauerstoff steht, Y für Schwefel steht und $R^3$ für Wasserstoff steht, und einer Verbindung mit der allgemeinen Formel (I), bei der X und Y beide für Schwefel stehen und $R^3$ für Wasserstoff steht;

d) wenn X für Schwefel steht, Y für Sauerstoff steht und $R^3$ für H steht,

i) ein Isothiocyanat mit der folgenden allgemeinen Formel (XXI):

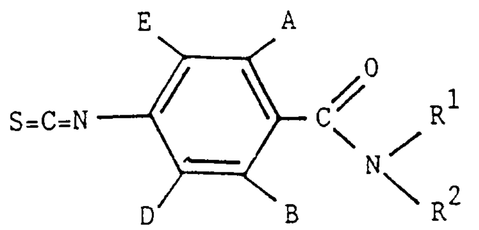
(XXI)

mit einem Organometallreagenz $R^4Li$ oder $R^4Mg$-hal in einem geeigneten Lösungsmittel bei einer Temperatur zwischen -78 °C und +25 °C umgesetzt wird; oder

ii) ein Säurechlorid (XXII):

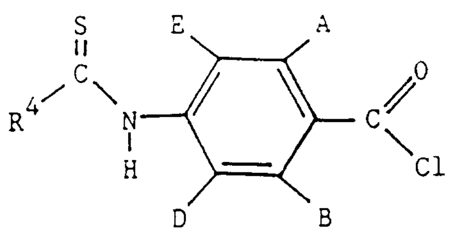
(XXII)

mit einem Amin $R^1R^2NH$ in der Gegenwart einer Base umgesetzt wird;
wobei A, B, D, E, $R^1$, $R^2$ und $R^4$ (sofern nichts anderes angegeben ist) die in Anspruch 1 angegebenen Bedeutungen haben, $R^8$ und $R^9$ beide für Methyl stehen, hal für Halogen steht und L für eine Austrittsgruppe steht.

**7.** Fungicide-Zusammensetzung, die eine fungicid wirksame Menge einer Verbindung nach Anspruch 1 und ein für Fungicide geeignetes Trägermittel oder Verdünnungsmittel enthält.

**8.** Verfahren zur Pilzbekämpfung, bei dem eine Verbindung nach Anspruch 1 oder eine Zusammensetzung nach Anspruch 7 auf Pflanzen, das Saatgut von Pflanzen oder auf den Standort der Pflanzen oder des Saatguts aufgebracht wird.

**Patentansprüche für folgenden Vertragsstaat : ES**

**1.** Fungicide Zusammensetzung, die bis zu 95 Gew.-% einer Verbindung mit der folgenden Formel (I) enthält:

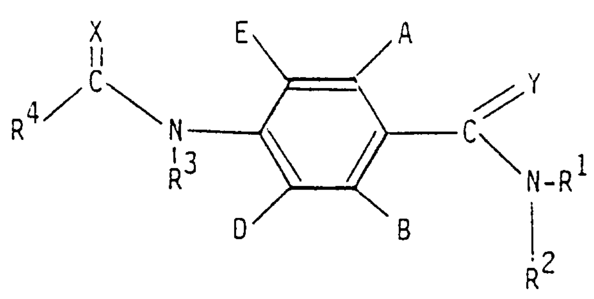
(I)

in der A und B unabhängig voneinander für folgendes stehen: H, Jod, Nitro, Cyano, $C_{1-4}$-Alkoxycarbonyl $C_{1-4}$-Alkoxy($C_{1-4}$)alkyl, $C_{1-4}$-Alkylthio($C_{1-4}$)Alkyl, Formyl, $C_{1-4}$-Alkylthio, $C_{1-4}$-Alkylcarbonyl, $-CR^5=NOR^6$ (wobei $R^5$ und $R^6$ unabhängig voneinander für H oder $C_{1-4}$-Alkyl stehen), $C_{2-4}$-Alkenyl

oder $C_{2-4}$-Alkinyl, mit der Maßgabe, daß A und B nicht beide für H stehen; D und E unabhängig voneinander für H oder Fluor stehen; $R^1$ für $C_{1-4}$-Alkyl oder $C_{1-4}$-Alkoxy steht; $R^2$ für $C_{1-4}$-Alkyl oder $C_{1-4}$-Alkoxy steht, oder in der $R^1$ und $R^2$ zusammen mit dem Stickstoffatom, an das sie gebunden sind, unter Bildung eines Morpholin-, Piperidin-, Pyrrolidin- oder Azetidin-Rings miteinander verbunden sind, der gegebenenfalls mit $C_{1-4}$-Alkyl substituiert ist; $R^3$ für H steht; $R^4$ für $R(CH_3)_2C$ steht, wobei R für Halogen, $C_{1-4}$-Alkyl oder $C_{1-4}$-Alkoxy steht; und X und Y unabhängig voneinander für Sauerstoff oder Schwefel stehen.

**2.** Zusammensetzung nach Anspruch 1, wobei der $R^4$ für $F(CH_3)_2C$ steht.

**3.** Zusammensetzung nach Anspruch 1, wobei A für Jod, Cyano, Nitro, $C_{1-4}$-Alkoxycarbonyl, $C_{1-4}$-Alkylcarbonyl, $C_{1-4}$-Alkoxy($C_{1-4}$)alkyl, $C_{1-4}$-Alkylthio, Formyl, $C_{1-4}$-Alkylthio($C_{1-4}$)alkyl oder $-CH=NOR^6$ steht (wobei $R^6$ für $C_{1-4}$-Alkyl steht); $R^1$ und $R^2$ für Alkyl stehen; $R^3$, B, D und E für H stehen; $R^4$ für $C_{2-6}$-Alkyl steht, das gegebenenfalls mit Halogen oder $C_{1-4}$-Alkoxy substituiert ist; und X und Y beide für Sauerstoff stehen.

**4.** Verfahren zur Herstellung einer Verbindung mit der folgenden Formel (I):

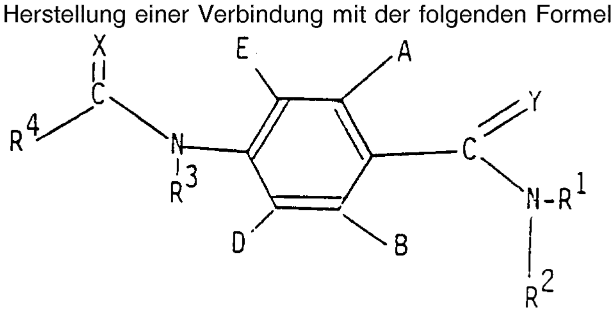

(I)

in der A und B unabhängig voneinander für folgendes stehen: H, Jod, Nitro, Cyano, $C_{1-4}$-Alkoxycarbonyl $C_{1-4}$-Alkoxy($C_{1-4}$)alkyl, $C_{1-4}$-Alkylthio($C_{1-4}$)Alkyl, Formyl, $C_{1-4}$-Alkylthio, $C_{1-4}$-Alkylcarbonyl, $-CR^5=NOR^6$ (wobei $R^5$ und $R^6$ unabhängig voneinander für H oder $C_{1-4}$-Alkyl stehen), $C_{2-4}$-Alkenyl oder $C_{2-4}$-Alkinyl, mit der Maßgabe, daß A und B nicht beide für H stehen; D und E unabhängig voneinander für H oder Fluor stehen; $R^1$ für $C_{1-4}$-Alkyl oder $C_{1-4}$-Alkoxy steht; $R^2$ für $C_{1-4}$-Alkyl oder $C_{1-4}$-Alkoxy steht, oder in der $R^1$ und $R^2$ zusammen mit dem Stickstoffatom, an das sie gebunden sind, unter Bildung eines Morpholin-, Piperidin-, Pyrrolidin- oder Azetidin-Rings miteinander verbunden sind, der gegebenenfalls mit $C_{1-4}$-Alkyl substituiert ist; $R^3$ für H steht; $R^4$ für $R(CH_3)_2C$ steht, wobei R für Halogen, $C_{1-4}$-Alkyl oder $C_{1-4}$-Alkoxy steht; und X und Y unabhängig voneinander für Sauerstoff oder Schwefel stehen, wobei bei dem Verfahren:

(a) für eine Verbindung, bei der A für Jod steht, $R^3$ für H steht und X und Y beide für Sauerstoff stehen, eine Verbindung mit der folgenden Formel (VIII):

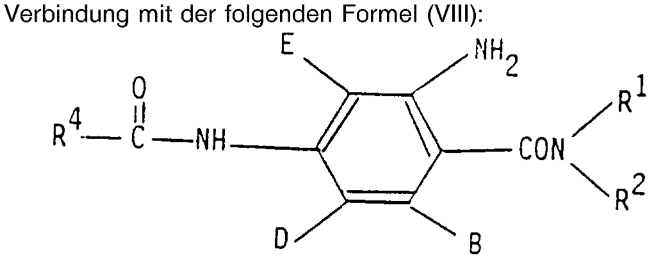

(VIII)

mit salpetriger Säure und einer Jodid-Quelle behandelt wird;

(b) für eine Verbindung, bei der A für Cyano oder $C_{1-4}$-Alkylthio steht, $R^3$ für H steht und X und Y beide für Sauerstoff stehen, eine Verbindung mit der folgenden Formel (IX):

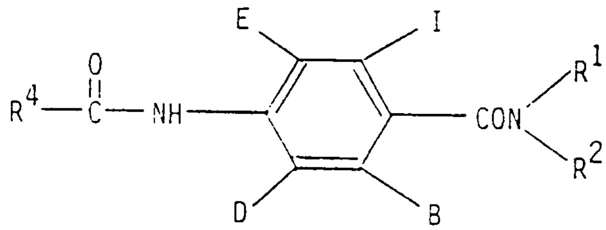

(IX)

mit einem Übergangsmetall-Salz eines Cyanids oder $C_{1-4}$-Alkanthiolats in einem geeigneten Lösungsmittel umgesetzt wird,

(c) wenn X und Y beide für Sauerstoff stehen und $R^3$ für H steht,

(i) eine Verbindung mit der folgenden allgemeinen Formel (X):

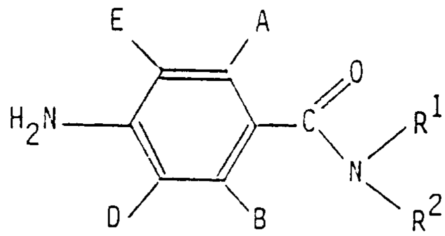

(X)

mit einem Säurechlorid $R^4COCl$ in einem geeigneten organischen Lösungsmittel in der Gegenwart einer Base umgesetzt wird;

(ii) eine Verbindung mit der folgenden allgemeinen Formel (XI):

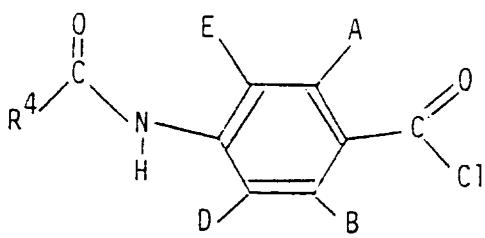

(XI)

mit einem Amin $R^1R^2NH$ in einem geeigneten organischen Lösungsmittel in der Gegenwart einer Base oder eines Überschusses $R^1R^2NH$ umgesetzt wird; oder

(iii) eine Verbindung mit der folgenden allgemeinen Formel (XII):

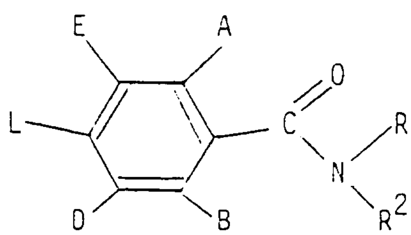

(XII)

mit einer Verbindung mit der allgemeinen Formel $R^4$-CO-$NH_2$ und einer Base umgesetzt wird;

(d) wenn X und Y beide für Sauerstoff stehen, $R^3$ für H steht und $R^4$ für die Gruppe (XV) steht:

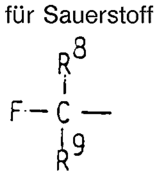

(XV)

(i) eine Verbindung mit der folgenden allgemeinen Formel (XVI):

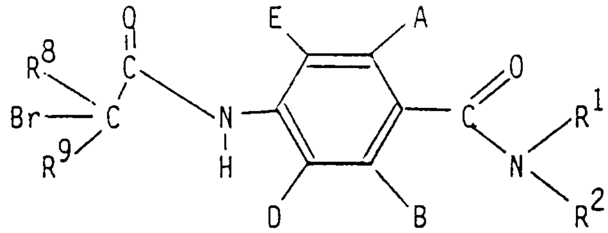

(XVI)

mit einem Fluorid-Übertragungsmittel in einem geeigneten Lösungsmittel behandelt wird;

(e) wenn X für Sauerstoff oder Schwefel steht, Y für Schwefel steht und $R^3$ für H steht,

(i) eine Verbindung mit der folgenden allgemeinen Formel (II):

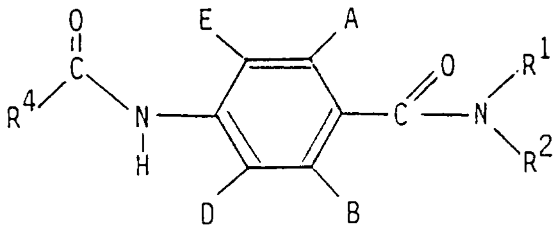

(II)

mit einem Thionierungsreagenz in einem geeigneten Lösungsmittel behandelt wird, und zwar entweder unter Bildung einer Verbindung mit der allgemeinen Formel (I), bei der X für Sauerstoff steht, Y für Schwefel steht und $R^3$ für Wasserstoff steht, oder eines Gemisches aus einer Verbindung mit der allgemeinen Formel (I), bei der X für Sauerstoff steht, Y für Schwefel steht und $R^3$ für Wasserstoff steht, und einer Verbindung mit der allgemeinen Formel (I), bei der X und Y beide für Schwefel stehen und $R^3$ für Wasserstoff steht;

(f) wenn X für Schwefel steht, Y für Sauerstoff steht und $R^3$ für H steht,

i) ein Isothiocyanat mit der folgenden allgemeinen Formel (XXI):

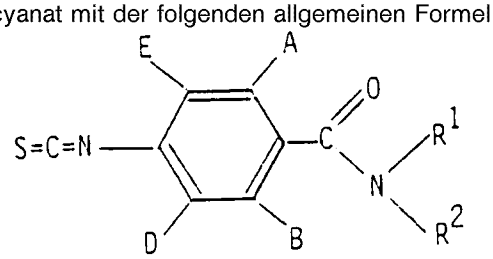

(XXI)

mit einem Organometallreagenz $R^4$Li oder $R^4$Mg-hal in einem geeigneten Lösungsmittel bei einer Temperatur zwischen -78 °C und +25 °C umgesetzt wird; oder

(ii) ein Säurechlorid (XXII):

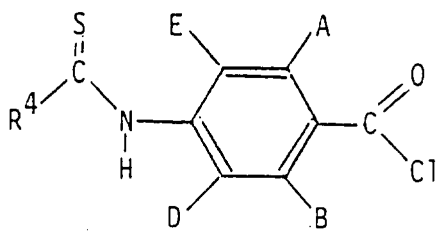

(XXII)

mit einem Amin $R^1R^2NH$ in der Gegenwart einer Base umgesetzt wird;

wobei $R^8$ und $R^9$ beide für Methyl stehen, hal für Halogen steht und L für eine Austrittsgruppe steht.

5. Verfahren nach Anspruch 4, wobei $R^4$ für $F(CH_3)_2C$ steht.

6. Verfahren nach Anspruch 4, wobei A für Jod, Cyano, Nitro, $C_{1-4}$-Alkoxycarbonyl, $C_{1-4}$-Alkylcarbonyl, $C_{1-4}$-Alkoxy($C_{1-4}$)alkyl, $C_{1-4}$-Alkylthio, Formyl, $C_{1-4}$-Alkylthio($C_{1-4}$)alkyl oder -CH=NOR$^6$ steht (wobei $R^6$ für $C_{1-4}$-Alkyl steht); $R^1$ und $R^2$ für Alkyl stehen; $R^3$, B, D und E für H stehen; $R^4$ für $C_{2-6}$-Alkyl steht, das gegebenenfalls mit Halogen oder $C_{1-4}$-Alkoxy substituiert ist; und X und Y beide für Sauerstoff stehen.

**Revendications**

**Revendications pour les Etats contractants suivants : AT, BE, CH, DE, DK, FR, GB, GR, IT, LI, LU, NL, SE**

1. Composé de formule (I) :

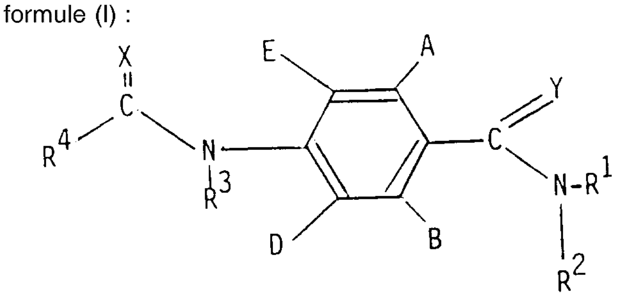

(I)

dans laquelle A et a représentent, indépendamment, H, un radical iodo, nitro, cyano, (alkoxy en $C_1$ à $C_4$)carbonyle, (alkoxy en $C_1$ à $C_4$)-(alkyle en $C_1$ à $C_4$), (alkylthio en $C_1$ à $C_4$)-(alkyle en $C_1$ à $C_4$), formyle, alkylthio en $C_1$ à $C_4$, (alkyle en $C_1$ à $C_4$)carbonyle, -CR$^5$=NOR$^6$ (où $R^5$ et $R^6$ représentent indépendamment H ou un radical alkyle en $C_1$ à $C_4$), alcényle en $C_2$ à $C_4$ ou alcynyle en $C_2$ à $C_4$, sous réserve que A et B ne représentent pas tous deux de l'hydrogène ; D et E représentent, indépendamment, de l'hydrogène ou un radical fluoro ; $R^1$ est un groupe alkyle en $C_1$ à $C_4$ ou alkoxy en $C_1$ à $C_4$ ; $R^2$ est un groupe alkyle en $C_1$ à $C_4$ ou alkoxy en $C_1$ à $C_4$, ou bien $R^1$ et $R^2$ forment, conjointement avec l'atome d'azote auquel ils sont attachés, un noyau de morpholine, pipéridine, pyrrolidine ou azétidine qui porte facultativement un substituant alkyle en $C_1$ à $C_4$ ; $R^3$ représente H ; $R^4$ est un groupe $R(CH_3)_2C$ où R est un radical halogéno, alkyle en $C_1$ à $C_4$ ou alkoxy en $C_1$ à $C_4$ ; et X et Y représentent indépendamment de l'oxygène ou du soufre.

2. Composé suivant la revendication 1, dans lequel $R^4$ est un groupe $F(CH_3)_2C$.

3. Composé suivant la revendication 1, dans lequel A est un radical iodo, cyano, nitro, (alkoxy en $C_1$ à $C_4$)carbonyle, (alkyle en $C_1$ à $C_4$)carbonyle, (alkoxy en $C_1$ à $C_4$)-(alkyle en $C_1$ à $C_4$), alkylthio en $C_1$ à $C_4$, formyle, (alkylthio en $C_1$ à $C_4$)-(alkyle en $C_1$ à $C_4$) ou -CH=NOR$^6$ (où $R^6$ est un radical alkyle en $C_1$ à $C_4$) ; $R^1$ et $R^2$ sont des groupes alkyle ; $R^3$, B, D et E représentent de l'hydrogène ; $R^4$ est un groupe alkyle en $C_2$ à $C_6$ facultativement substitué avec un halogène ou un radical alkoxy en $C_1$ à $C_4$ ; et X et Y sont tous deux de l'oxygène.

4. Procédé de production d'un composé suivant la revendication 1, dans lequel A est un radical iodo, $R^3$ est de l'hydrogène et X et Y sont tous deux de l'oxygène, qui comprend le traitement d'un composé de formule (VIII) :

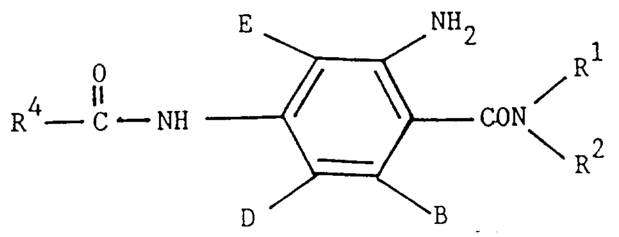

(VIII)

avec de l'acide nitreux et une source d'iodure ; B, D, E, $R^1$, $R^2$ et $R^4$ ayant les définitions indiquées

dans la revendication 1.

**5.** Procédé de production d'un composé suivant la revendication 1, dans lequel A est un groupe cyano ou alkylthio en $C_1$ à $C_4$, $R^3$ est de l'hydrogène et X et Y sont tous deux de l'oxygène, qui comprend la réaction d'un composé de formule (IX) :

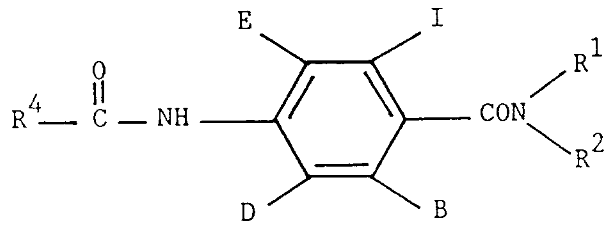

(IX)

avec un sel de métal de transition d'un cyanure ou d'un alcanethiolate en $C_1$ à $C_4$ dans un solvant convenable ; B, D, E, $R^1$, $R^2$ et $R^4$ ayant les définitions indiquées dans la revendication 1.

**6.** Procédé de production d'un composé suivant la revendication 1,

a) lorsque X et Y sont tous deux de l'oxygène et $R^3$ est de l'hydrogène

i) par réaction d'un composé de formule générale (X) :

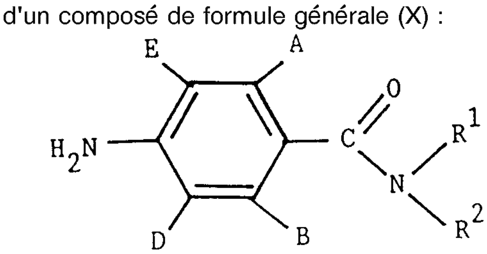

(X)

avec un chlorure d'acide $R^4COCl$ dans un solvant organique convenable en présence d'une base ;

ii) par réaction d'un composé de formule générale (XI) :

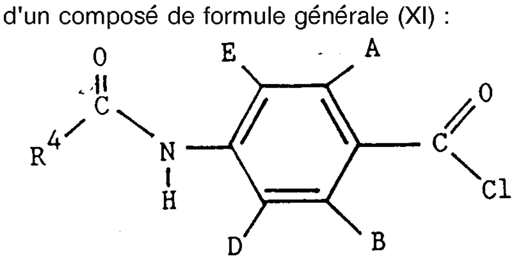

(XI)

avec une amine $R^1R^2NH$ dans un solvant organique convenable en présence d'une base ou de $R^1R^2NH$ en excès ; ou bien

iii) par réaction d'un composé de formule générale (XIII):

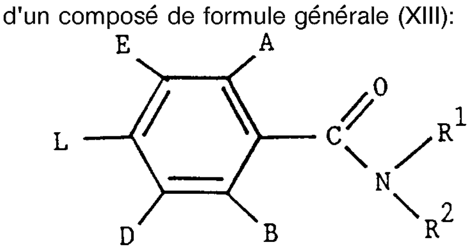

(XII)

avec un compose de formule générale $R^4$-CO-$NH_2$ et une base ;

b) lorsque X et Y sont tous deux de l'oxygène, $R^3$ est de l'hydrogène et $R^4$ est le groupe (XV) :

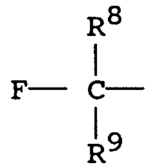

(XV)

i) par traitement d'un composé de formule générale (XVI) :

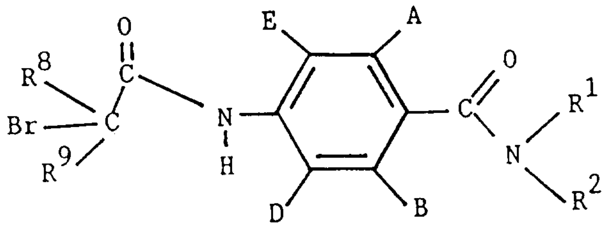

(XVI)

avec un réactif de transfert du type d'un fluorure dans un solvant convenable ;

c) lorsque X est de l'oxygène ou du soufre, Y est du soufre et $R^3$ est de l'hydrogène,

i) par traitement d'un composé de formule générale (II) :

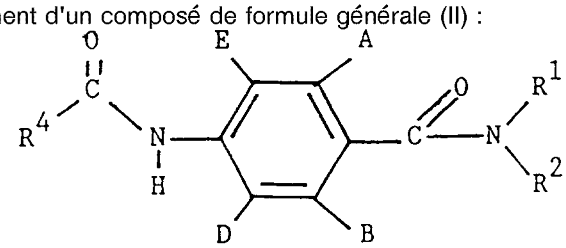

(II)

avec un agent de thionation dans un solvant convenable pour former un composé de formule générale (I) dans laquelle X est de l'oxygène, Y est du soufre et $R^3$ est de l'hydrogène ou un mélange d'un composé de formule générale (I) dans laquelle X est de l'oxygène, Y est du soufre et $R^3$ est de l'hydrogène et d'un composé de formule générale (I) dans laquelle X et Y sont tous deux du soufre et $R^3$ est de l'hydrogène ;

d) lorsque X est du soufre, Y est de l'oxygène et $R^3$ est de l'hydrogène,

i) par réaction d'un isothiocyanate de formule générale (XXI) :

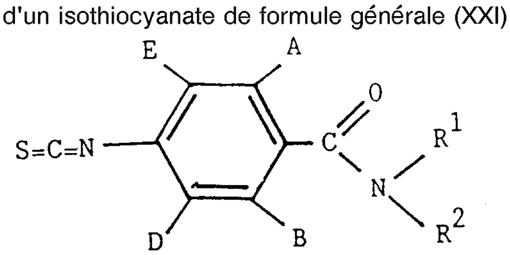

(XXI)

avec un réactif organométallique $R^4Li$ ou $R^4Mg$-hal dans un solvant convenable à une température comprise entre -78 °C et +25 °C ; ou bien

ii) par réaction d'un chlorure d'acide (XXII) :

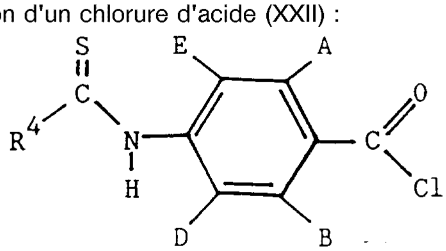

(XXII)

avec une amine $R^1R^2NH$ en présence d'une base ;

formules dans lesquelles A, B, D, E, $R^1$, $R^2$ et $R^4$ (sauf spécification contraire) ont les définitions indiquées dans la revendication 1, $R^8$ et $R^9$ sont tous deux des groupes méthyle, hal est un halogène et L est un groupe partant.

7. Composition fongicide, comprenant une quantité à effet fongicide d'un composé suivant la revendication 1 et un support ou diluant approprié acceptable du point de vue fongicide.

8. Procédé pour combattre des champignons, qui comprend l'application à des plantes, aux graines de plantes ou sur le site de plantes ou de graines, d'un composé suivant la revendication 1 ou d'une composition suivant la revendication 7.

**Revendication pour l'Etat contractant suivant : ES**

1. Composition fongicide, comprenant jusqu'à 95 % en poids d'un composé de formule (I) :

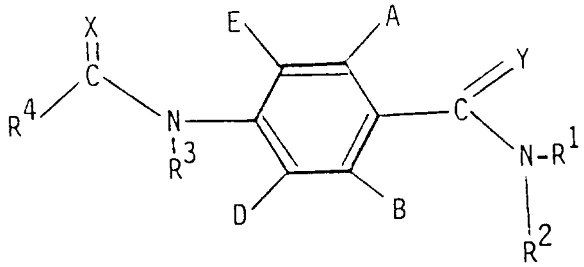

(I)

dans laquelle A et a représentent, indépendamment, H, un radical iodo, nitro, cyano, (alkoxy en $C_1$ à $C_4$)carbonyle, (alkoxy en $C_1$ à $C_4$)-(alkyle en $C_1$ à $C_4$), (alkylthio en $C_1$ à $C_4$)-(alkyle en $C_1$ à $C_4$), formyle, alkylthio en $C_1$ à $C_4$, (alkyle en $C_1$ à $C_4$)carbonyle, $-CR^5=NOR^6$ (où $R^5$ et $R^6$ représentent indépendamment H ou un radical alkyle en $C_1$ à $C_4$), alcényle en $C_2$ à $C_4$ ou alcynyle en $C_2$ à $C_4$, sous réserve que A et B ne représentent pas tous deux de l'hydrogène ; D et E représentent, indépendamment, de l'hydrogène ou un radical fluoro ; $R^1$ est un groupe alkyle en $C_1$ à $C_4$ ou alkoxy en $C_1$ à $C_4$ ; $R^2$ est un groupe alkyle en $C_1$ à $C_4$ ou alkoxy en $C_1$ à $C_4$, ou bien $R^1$ et $R^2$ forment, conjointement avec l'atome d'azote auquel ils sont attachés, un noyau de morpholine, pipéridine, pyrrolidine ou azétidine qui porte facultativement un substituant alkyle en $C_1$ à $C_4$ : $R^3$ représente H ; $R^4$ est un groupe $R(CH_3)_2C$ où R est un radical halogéno, alkyle en $C_1$ à $C_4$ ou alkoxy en $C_1$ à $C_4$ ; et X et Y représentent indépendamment de l'oxygène ou du soufre.

2. Composition suivant la revendication 1, dans laquelle $R^4$ est un groupe $F(CH_3)_2C$.

3. Composition suivant la revendication 1, dans laquelle A représente un radical iodo, cyano, nitro, (alkoxy en $C_1$ à $C_4$)carbonyle, (alkyle en $C_1$ à $C_4$)carbonyle, (alkoxy en $C_1$ à $C_4$)-(alkyle en $C_1$ à $C_4$), alkylthio en $C_1$ à $C_4$, formyle, (alkylthio en $C_1$ à $C_4$)-(alkyle en $C_1$ à $C_4$) ou $-CH=NOR^6$ (où $R^6$ est un radical alkyle en $C_1$ à $C_4$) ; $R^1$ et $R^2$ sont des groupes alkyle ; $R^3$, B, D et E représentent de l'hydrogène ; $R^4$ est un groupe alkyle en $C_2$ à $C_6$ facultativement substitué avec un halogène ou un radical alkoxy en $C_1$ à $C_4$ ; et X et Y sont tous deux de l'oxygène.

4. Procédé de production d'un composé de formule (I) :

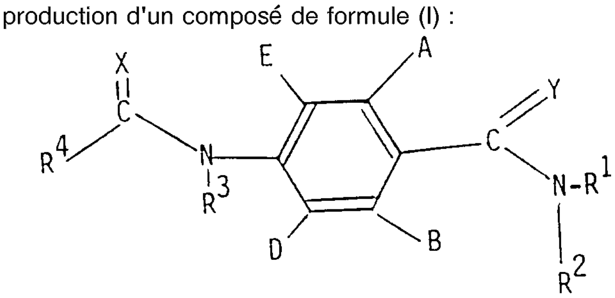

(I)

dans laquelle A et B représentent, indépendamment, H, un radical iodo, nitro, cyano, (alkoxy en $C_1$ à $C_4$)carbonyle, (alkoxy en $C_1$ à $C_4$)-(alkyle en $C_1$ à $C_4$), (alkylthio en $C_1$ à $C_4$)-(alkyle en $C_1$ à $C_4$),

formyle, alkylthio en $C_1$ à $C_4$, (alkyle en $C_1$ à $C_4$)carbonyle, $-CR^5=NOR^6$ (où $R^5$ et $R^6$ représentent indépendamment H ou un radical alkyle en $C_1$ à $C_4$), alcényle en $C_2$ à $C_4$ ou alcynyle en $C_2$ à $C_4$, sous réserve que A et B ne représentent pas tous deux de l'hydrogène ; D et E représentent, indépendamment, de l'hydrogène ou un radical fluoro ; $R^1$ est un groupe alkyle en $C_1$ à $C_4$ ou alkoxy en $C_1$ à $C_4$ ; $R^2$ est un groupe alkyle en $C_1$ à $C_4$ ou alkoxy en $C_1$ à $C_4$, ou bien $R^1$ et $R^2$ forment, conjointement avec l'atome d'azote auquel ils sont attachés, un noyau de morpholine, pipéridine, pyrrolidine ou azétidine qui porte facultativement un substituant alkyle en $C_1$ à $C_4$ ; $R^3$ représente H ; $R^4$ est un groupe $R(CH_3)_2C$ où R est un radical halogéno, alkyle en $C_1$ à $C_4$ ou alkoxy en $C_1$ à $C_4$ ; et X et Y représentent indépendamment de l'oxygène ou du soufre ; ce procédé comprenant :

(a) pour un composé dans lequel A est un radical iodo, $R^3$ est de l'hydrogène et X et Y sont tous deux de l'oxygène, le traitement d'un composé de formule (VIII) :

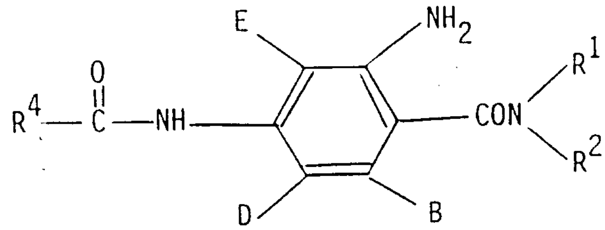

(VIII)

avec de l'acide nitreux et une source iodure ;

(b) pour un composé dans lequel A est un radical cyano ou alkylthio en $C_1$ à $C_4$, $R^3$ est de l'hydrogène et X et Y sont tous deux de l'oxygène, la réaction d'un composé de formule (IX) :

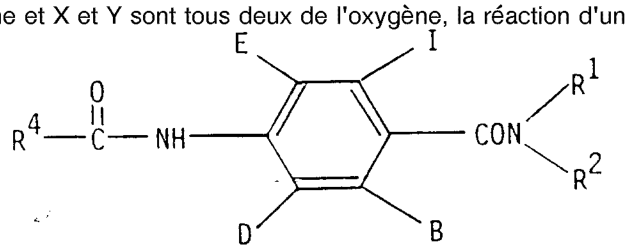

(IX)

avec un sel de métal de transition d'un cyanure ou d'un alcanethiolate en $C_1$ à $C_4$ dans un solvant convenable ;

(c) lorsque X et Y sont tous deux de l'oxygène et $R^3$ est de l'hydrogène ;

i) par réaction d'un composé de formule générale (X) :

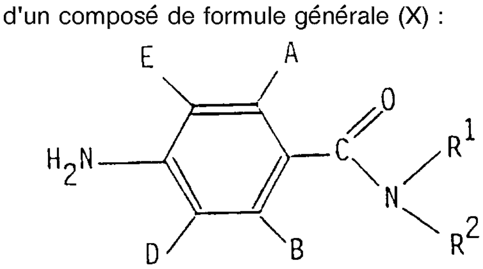

(X)

avec un chlorure d'acide $R^4COCl$ dans un solvant organique convenable en présence d'une base ;

ii) par réaction d'un composé de formule générale (XI) :

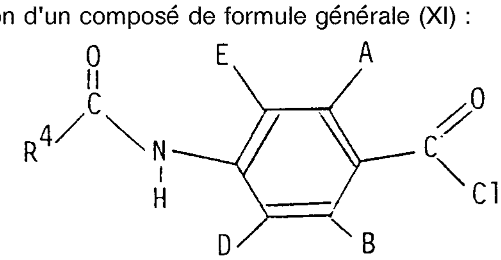

(XI)

avec une amine $R^1R^2NH$ dans un solvant organique convenable en présence d'une base ou de $R^1R^2NH$ en excès ; ou

iii) par réaction d'un composé de formule générale (XII) :

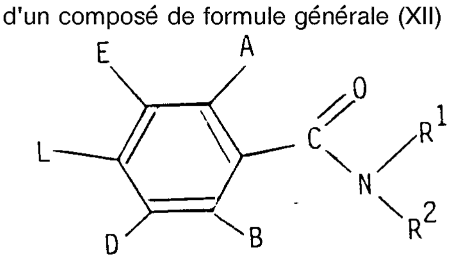

(XII)

avec un composé de formule générale $R^4$-CO-$NH_2$ et une base ;

d) lorsque X et Y sont tous deux de l'oxygène, $R^3$ est de l'hydrogène et $R^4$ est le groupe (XV) :

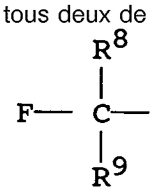

**(XV)**

i) par traitement d'un composé de formule générale (XVI) :

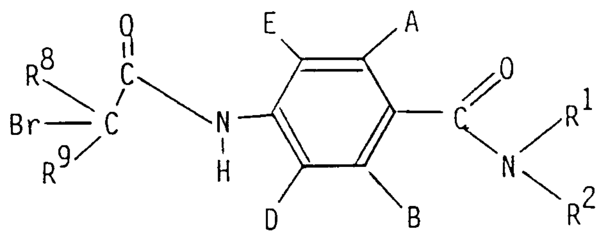

(XVI)

avec un réactif de transfert du type d'un fluorure dans un solvant convenable ;

e) lorsque X est de l'oxygène ou du soufre, Y est du soufre et $R^3$ est de l'hydrogène, par traitement d'un composé de formule générale (II) :

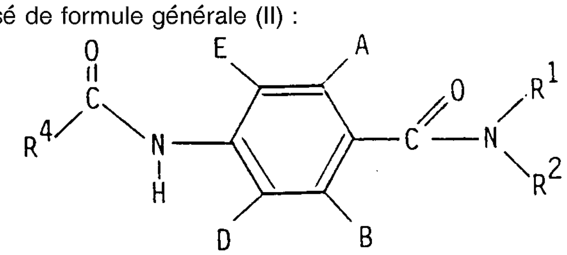

(II)

avec un réactif de thionation dans un solvant convenable pour former un composé de formule générale (I) dans laquelle X est de l'oxygène, Y est du soufre et $R^3$ est de l'hydrogène ou un mélange d'un composé de formule générale (I) dans laquelle X est de l'oxygène, Y est du soufre et $R^3$ est de l'hydrogène, et un composé de formule générale (I) dans laquelle X et Y sont tous deux du soufre et $R^3$ est de l'hydrogène ;

f) lorsque X est du soufre, Y est de l'oxygène et $R^3$ est de l'hydrogène,

i) par réaction d'un isothiocyanate de formule générale (XXI) :

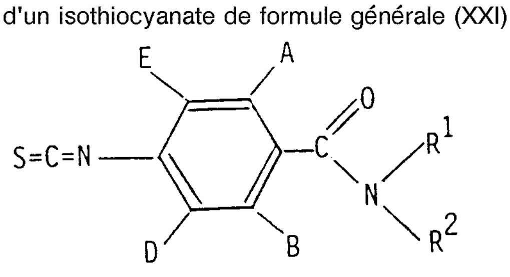

(XXI)

avec un réactif organométallique $R^4Li$ ou $R^4Mg$-hal dans un solvant convenable à une température comprise entre -78 ° C et +25 ° C ; ou bien

ii) par réaction d'un chlorure d'acide (XXII) :

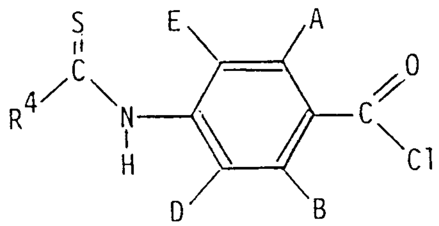

(XXII)

avec une amine $R^1R^2NH$ en présence d'une base ;

$R^8$ et $R^9$ étant tous deux des groupes méthyle, hal représentant un halogène et L étant un groupe partant.

**5.** Procédé suivant la revendication 4, dans lequel $R^4$ est un groupe $F(CH_3)_2C$.

**6.** Procédé suivant la revendication 4, dans lequel A est un radical iodo, cyano, nitro, (alkoxy en $C_1$ à $C_4$)-carbonyle, (alkyle en $C_1$ à $C_4$)carbonyle, (alkoxy en $C_1$ à $C_4$)-(alkyle en $C_1$ à $C_4$), alkylthio en $C_1$ à $C_4$, formyle, (alkylthio en $C_1$ à $C_4$)-(alkyle en $C_1$ à $C_4$) ou $-CH=NOR^6$ (où $R^6$ est un radical alkyle en $C_1$ à $C_4$) ; $R^1$ et $R^2$ sont des groupes alkyle ; $R^3$, B, D et E représentent de l'hydrogène ; $R^4$ est un groupe alkyle en $C_2$ à $C_4$ facultativement substitué avec un halogène ou un radical alkoxy en $C_1$ à $C_4$ ; et X et Y sont tous deux de l'oxygène.